(19) Europäisches Patentamt
European Patent Office
Office européen des brevets

(11) **EP 4 082 623 A1**

(12) **EUROPEAN PATENT APPLICATION**
published in accordance with Art. 153(4) EPC

(43) Date of publication:
**02.11.2022 Bulletin 2022/44**

(21) Application number: **20907520.9**

(22) Date of filing: **28.12.2020**

(51) International Patent Classification (IPC):
*A61Q 13/00* (2006.01)   *A61K 9/10* (2006.01)
*A61K 9/50* (2006.01)   *A61K 8/04* (2006.01)
*A61K 8/11* (2006.01)   *A61K 8/25* (2006.01)
*A61K 8/34* (2006.01)   *A61K 8/891* (2006.01)
*A61K 47/02* (2006.01)   *A61K 47/10* (2017.01)
*A61K 47/34* (2017.01)   *B01J 13/14* (2006.01)
*C11B 9/00* (2006.01)

(52) Cooperative Patent Classification (CPC):
**A61K 8/04; A61K 8/11; A61K 8/25; A61K 8/34;**
**A61K 8/891; A61K 9/10; A61K 9/50; A61K 47/02;**
**A61K 47/10; A61K 47/34; A61Q 13/00;**
**B01J 13/14; C11B 9/00**

(86) International application number:
**PCT/JP2020/049255**

(87) International publication number:
**WO 2021/132726 (01.07.2021 Gazette 2021/26)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **27.12.2019 JP 2019239906**

(71) Applicant: **Kao Corporation**
**Chuo-ku**
**Tokyo 103-8210 (JP)**

(72) Inventors:
• **SAWADA, Risa**
**Wakayama-shi, Wakayama 640-8580 (JP)**

• **MOROFUJI, Tatsuya**
**Wakayama-shi, Wakayama 640-8580 (JP)**
• **YAMAZAKI, Daisuke**
**Wakayama-shi, Wakayama 640-8580 (JP)**
• **KOGA, Yoshito**
**Wakayama-shi, Wakayama 640-8580 (JP)**
• **ICHIKAWA, Tomohiko**
**Tokyo 131-8501 (JP)**
• **KIYOFUJI, Harumi**
**Tokyo 131-8501 (JP)**

(74) Representative: **Hoffmann Eitle**
**Patent- und Rechtsanwälte PartmbB**
**Arabellastraße 30**
**81925 München (DE)**

(54) **SILICA MICROCAPSULES**

(57)    The present invention relates to silica microcapsules, each of which includes a shell, and a core containing one or more organic compounds inside the shell, a softener composition containing the silica microcapsules, and a method of producing the silica microcapsules. In the silica microcapsules, the shell contains silica as a constituent component, and the organic compound contains a primary alcohol.

EP 4 082 623 A1

**Description**

FIELD OF THE INVENTION

The present invention relates to silica microcapsules.

BACKGROUND OF THE INVENTION

In a broad business field such as cosmetics, drugs and medicines, general household goods, and printing, various microcapsules encapsulating fragrances or physiologically active substances therein have been developed and utilized. As shells constituting microcapsules, aminoplast resins such as a melamine resin, or a polyurea/urethane resin have been used. However, microcapsules are inevitably discharged into the environment, and have recently become one of factors of substances of concern called microplastics. Thus, it is required to develop microcapsules with high environmental friendliness in replacement of aminoplast resins.

Among them, silica microcapsules (hereinafter, also called "silica capsules") having shells whose constituent component is silica are attracting attention as a material that may be expected to be environmentally friendly. However, a high denseness of the shells is required for long-term stable blending in an oil agent-containing formulation such as cosmetics, liquid detergents, and fabric softeners, or a high-concentration surfactant-containing formulation. Further, a formulation has various pH values and viscosities according to its purpose, and thus an ability to blend with formulations having a wide range of physical characteristics is also required. Therefore, various silica capsules have heretofore been studied.

For example, JP H4-265149A (PTL 1) aims at providing a micro-encapsulated body or the like containing a hydrophobic substance, and describes a micro-encapsulated body that uses colloidal silica or fumed silica, which contains an outer silica layer containing a hydrophobic substance such as a fragrance.

Meanwhile, a core-shell type microcapsule, in which a shell has silica as a constituent component, and an oil-soluble component or the like is a core component, has also been studied.

US 9532933 (PTL 2) aims at obtaining a silica capsule particle composition having sturdy shells and describes a microcapsule particle composition or the like which has a core material encapsulated within a microcapsule shell, and describes silica capsule particles are formed using a fragrance as a core material, and a cationic surfactant as an emulsifier, and then, the silica capsule particles are treated with polyethyleneimine, etc.

J P 2009-542667 (PTL 3) aims at obtaining capsules that are more resistant to diffusion or leaching of an oil phase from microcapsules and describes a method of preparing microcapsules having a sunscreen component or the like as a core, and describes tetraalkoxysilane is polymerized through ex-situ (extra system) emulsion polymerization by using a cationic surfactant and a nonionic surfactant in combination.

JP 2012-501849 (PTL 4) aims at improving the stability of the aqueous suspension of the silicate shell microcapsules, and describes a method of adding a colloidal silicate sequestering agent to an aqueous suspension of silicate shell microcapsules and colloidal silicate particles.

JP 2015-128762A (PTL 5) aims at enhancing the denseness and strength of the shells and describes a method of producing microcapsules having a core made of an organic compound such as a fragrance, a first shell that encloses the core, and a second shell that encloses the first shell, and describes the shells of the microcapsules are formed through two sol-gel reaction steps.

SUMMARY OF THE INVENTION

The present invention relates to silica microcapsules, each of which includes a shell, and a core containing one or more organic compounds inside the shell, wherein the silica microcapsules, the shell contains silica as a constituent component, and the organic compound contains a primary alcohol.

DETAILED DESCRIPTION OF THE INVENTION

Here, a primary alcohol is widely used as an active ingredient for cosmetics, drugs and medicines or the like, or used in compounded fragrances as a soft and low-threshold scent ingredient, and thus is an important ingredient as an encapsulated component of microcapsules. In general, an alcohol-based fragrance is frequently used for compounded fragrances because it has a softer scent than an ester-based fragrance and a low-threshold value. In particular, the primary alcohol is preferably used because it is excellent in the scent, and may give a fresh scent and thus has a high palatability.

Therefore, from the viewpoint of improving the degree of freedom of blending with a formulation in application, and controlling the particle size distribution, it is also required to reduce the particle size of a silica capsule. When the shells of the silica capsules are formed by a sol-gel reaction, there is a step of emulsifying an aqueous phase component containing a surfactant, and an oil phase component containing an encapsulated organic compound and raw material silica (silica precursor). Since the particle size of the obtained silica capsule also depends on the particle size of the emulsified droplet of the emulsified liquid obtained by this emulsification, it is also required to form fine emulsified droplets in a shorter time so as to improve the production efficiency of silica capsules having reduced particle sizes.

The present inventors have found that the particle size of a silica capsule may be unexpectedly reduced by using a primary alcohol as an encapsulated component, and have completed the present invention.

The present invention relates to silica microcapsules which encapsulate an organic compound containing a primary alcohol, a softener composition containing the silica microcapsules, and a method of producing the silica microcapsules, in which the production efficiency of silica capsules with reduced particle sizes is excellent.

The present inventors have found that it is possible to obtain silica microcapsules with reduced particle sizes by core-shell type silica microcapsules, each of which has a core that contains a primary alcohol-containing organic compound, and a shell that contains silica as a constituent component.

That is, the present invention relates to [1] to [4] below.

(1)A silica microcapsule, which includes a shell, and a core containing one or more organic compounds inside the shell,

wherein the silica microcapsule, the shell contains silica as a constituent component, and
the organic compound contains a primary alcohol.

(2) A softener composition containing the silica microcapsule according to the above (1).
(3) A method of producing a silica microcapsule, which includes a shell, and a core containing one or more organic compounds inside the shell,

wherein the shell contains silica as a constituent component,
the organic compound contains a primary alcohol, and
the method includes the following step I.
Step I: subjecting an emulsified liquid obtained by emulsifying an aqueous phase component containing a cationic surfactant and an oil phase component containing a primary alcohol-containing organic compound and tetraalkoxysilane, to a sol-gel reaction under an acidic condition, thereby forming a silica capsule that has a core, and a shell whose constituent component is silica, to obtain a water dispersion containing the silica capsule.

(4) A method of producing a silica microcapsule, which includes a shell, and a core containing one or more organic compounds inside the shell,

wherein the shell contains silica as a constituent component,
the organic compound contains a primary alcohol, and
the method includes the following steps 1 and 2.

Step 1: subjecting an emulsified liquid obtained by emulsifying an aqueous phase component containing a cationic surfactant and an oil phase component containing an organic compound and tetraalkoxysilane, to a sol-gel reaction under an acidic condition, thereby forming a silica microcapsule (1) that has a core, and a first shell whose constituent component is silica, to obtain a water dispersion containing the silica microcapsule (1).
Step 2: further adding tetraalkoxysilane to the silica microcapsule (1)-containing water dispersion obtained in the step 1, and performing a sol-gel reaction, thereby forming a silica microcapsule having a second shell that encloses the first shell.

According to the present invention, it is possible to provide silica microcapsules which encapsulate an organic compound containing a primary alcohol, a softener composition containing the silica microcapsules, and a method of producing the silica microcapsules, in which the production efficiency of silica capsules with reduced particle sizes is excellent.

[Silica microcapsules]

The silica microcapsule (silica capsule) of the present invention is a silica capsule having a shell, and a core containing one or more organic compounds inside the shell, whereinthe shell contains silica as a constituent component, and the organic compound contains a primary alcohol.

In the present specification, the long-term retention of the encapsulated organic compound containing the primary alcohol is also referred to as "long-term retention." Further, the production efficiency of silica capsules with reduced particle sizes is also simply referred to as "production efficiency."

According to the present invention, a silica capsule which encapsulates an organic compound containing a primary alcohol can be obtained, and further the production efficiency of the silica capsules can be improved. The reason is not clear, but is thought to be as follows.

Meanwhile, the silica capsule of the present invention contains the primary alcohol as an encapsulated component. Thus, when an emulsified droplet has a reduced particle size, the area of an oil-water interface which is a shell forming site of the silica capsule is increased with respect to the amount of the encapsulated component. Then, the amount of surplus silica precursor not contributing to shell formation can be suppressed, and the denseness and strength of the shell can be increased, and thus, it is thought that it is possible to obtain the silica capsule which encapsulates the organic compound containing the primary alcohol. Then, it is thought that when the shell is broken in response to various stimulating factors, the delivery performance of the primary alcohol can be satisfactorily exhibited, and the retention and release of the encapsulated component can be controlled.

Further, in general, in the production of the silica capsule, the relationship between an emulsification time and a median diameter $D_{50}$ of emulsified droplets depends on the production scale and a stirring unit used for preparing an emulsified liquid, but there is a limitation in performance improvement of the stirring unit. In the present invention, since the encapsulated organic compound contains the primary alcohol, it is thought that it is possible to improve the production efficiency of silica capsules with reduced particle sizes probably because fine emulsified droplets can be efficiently formed in a shorter time when an oil phase component containing the primary alcohol and an aqueous phase component are emulsified.

<Core>

The core of the silica capsule of the present invention contains one or more organic compounds.

The organic compound contains a primary alcohol from the viewpoint of reducing the particle size of the silica capsule.

From the same viewpoint as above, the number of carbon atoms of the primary alcohol is preferably 4 or more, more preferably 6 or more, further preferably 8 or more, and is preferably 18 or less, more preferably 16 or less, further preferably 14 or less, still more preferably 12 or less.

An index of the hydrophilicity or hydrophobicity of the primary alcohol can be a cLogP value, which is a calculated value of a common logarithm "LogP" of a partition coefficient P (n-octanol/water) between n-octanol and water. Here, the cLogP value is "LogP (cLogP)" calculated by the method described in A. Leo Comprehensive Medicinal Chemistry, Vol.4 C. Hansch, P. G. Sammens, J. B Taylor and C. A. Ramsden, Eds., P.295, Pergamon Press, 1990, and is a cLogP value calculated by a program CLOGP v4.01.

The cLogP value of the primary alcohol is preferably 1.0 or more, more preferably 2.0 or more, further preferably 3.0 or more, and is preferably 7.0 or less, more preferably 6.5 or less, further preferably 6.0 or less, still more preferably 5.5 or less.

From the viewpoint of satisfactorily exhibiting the delivery performance of the primary alcohol, it is desirable that the primary alcohol is one or more selected from the group consisting of fragrances (fragrance components in compounded fragrances), antibacterial agents, preservatives, repellents (for example, a pest repellent), and active pharmaceutical ingredients.

In the present invention, the primary alcohol is preferably a fragrance component. Even in a case where the silica capsules of the present invention are contained in a softener composition or the like, by being encapsulated as an aromatic component in the core, the primary alcohol is excellent in the delivery characteristic, and the fragrance releasing property based on pressure application. Thus, it is possible to enjoy the effect caused by the scent peculiar to the primary alcohol.

Specific examples of the primary alcohol include, for example, primary alcohols, such as linear saturated aliphatic primary alcohols such as 1-decanol, 1-undecanol, and 1-dodecanol (alcohol C-12); linear unsaturated aliphatic primary alcohols such as cis-3-hexenol; linear or branched aliphatic primary alcohols such as branched saturated aliphatic primary alcohols such as tetrahydrogeraniol; aliphatic primary alcohols containing a saturated or unsaturated ring structure such as 4-isopropylcyclohexanemethanol, and SANDALMYSORE CORE; terpene-based primary alcohols such as geraniol, nellol, and citronellol; and aromatic primary alcohols such as 2-phenylethylalcohol, cinnamyl alcohol, benzyl alcohol, 6-phenyl-1-hexanol, and Pamplefleur.

Among them, from the viewpoint of reducing the particle size of the silica capsule, the primary alcohol is preferably one or more selected from the group consisting of terpene-based primary alcohols, linear or branched aliphatic primary alcohols, and aromatic primary alcohols, more preferably one or more selected from the group consisting of terpene-based primary alcohols and aromatic primary alcohols. Specifically, the terpene-based primary alcohol is preferably geraniol, citronellol, or nellol, the aliphatic primary alcohol is preferably alcohol C-12, tetrahydrogeraniol, or cis-3-hexenol, and the aromatic primary alcohol is 2-phenylethylalcohol, 6-phenyl-1-hexanol, or benzyl alcohol.

Further, from the viewpoint of prescription flexibility as a fragrance, the primary alcohol is preferably one or more selected from the group consisting of geraniol, citronellol, nellol, 2-phenylethylalcohol, and tetrahydrogeraniol.

The primary alcohol may be used alone or in combination of two or more thereof. When two or more primary alcohols are used, the cLogP value of the primary alcohols contained in the encapsulated organic compound can be obtained by multiplying respective cLogP values of the primary alcohols by respective volume ratios of the primary alcohols, and adding up these.

The organic compound may contain components other than the primary alcohol.

The other component is preferably one or more selected from the group consisting of a fragrance, a fragrance precursor, an oil agent (for example, moisturizer), an antioxidant, a cold sense agent, a dye, a pigment, silicone, a solvent, and an oil-soluble polymer, besides the primary alcohol, is more preferably one or more selected from the group consisting of a fragrance, a fragrance precursor, an oil agent, an antioxidant, and a solvent besides the primary alcohol, and is further preferably one or more selected from the group consisting of a fragrance and a fragrance precursor besides the primary alcohol.

Examples of the fragrance precursor include a compound that releases a fragrance component by reacting with water, and a compound that releases a fragrance component by reacting with light.

Examples of the compound that releases a fragrance component by reacting with water include a silicic acid ester compound containing an alkoxy component derived from fragrance alcohol, a fatty acid ester compound containing an alkoxy component derived from fragrance alcohol, an acetal compound or a hemiacetal compound obtained through a reaction between a carbonyl component derived from fragrance aldehyde or fragrance ketone and an alcohol compound, a Schiff base compound obtained through a reaction between a carbonyl component derived from fragrance aldehyde or fragrance ketone and a primary amine compound, and a hemiaminal compound or a hydrazone compound obtained through a reaction between a carbonyl component derived from fragrance aldehyde or fragrance ketone and a hydrazine compound.

Examples of the compound that releases a fragrance component by reacting with light include a 2-nitrobenzylether compound containing an alkoxy component derived from fragrance alcohol, an $\alpha$-keto ester compound containing a carbonyl component derived from fragrance aldehyde or fragrance ketone, and a coumaric acid ester compound containing an alkoxy component derived from fragrance alcohol. These fragrance precursors may be used, for example, as a polymer such as a product of reaction between some carboxy groups of polyacrylic acid and fragrance alcohol.

From the viewpoint of increasing the encapsulation rate, and the viewpoint of improving the long-term retention, the organic compound preferably has an appropriate hydrophobicity. As an index indicating the hydrophilicity or hydrophobicity of the organic compound, the cLogP value can be used.

When the organic compound is composed of a plurality of constituent components, the cLogP value of the organic compound can be obtained by multiplying respective cLogP values of the constituent components by respective volume ratios of the constituent components, and adding up these.

The cLogP value of the organic compound is preferably 1.0 or more, more preferably 2.0 or more, further preferably 3.0 or more, still more preferably 4.0 or more, and is preferably 30 or less, more preferably 20 or less, further preferably 10 or less, still more preferably 7.0 or less.

When the cLogP value of the organic compound is 1.0 or more, in a sol-gel reaction using oil droplets in water, which will be described below, the encapsulation rate of the organic compound within the obtained silica capsule is improved. Further, even in a case in which the organic compound is composed of a plurality of fragrance components, like a fragrance composition, similarly, when the cLogP value of the fragrance composition is 1.0 or more, the encapsulation rate of the fragrance composition within the silica capsule obtained through a sol-gel reaction can be improved.

In the present invention, not only an organic compound having a high oil-water interfacial tension but also an organic compound having a relatively low oil-water interfacial tension can be encapsulated as the core of the silica capsule.

From the viewpoint of ease in forming a core-shell type silica capsule, the oil-water interfacial tension of the organic compound encapsulated as the core is preferably 3 mN/m or more, more preferably 4 mN/m or more, further preferably 5 mN/m or more, still more preferably 7 mN/m or more, and is preferably 40 mN/m or less, more preferably 30 mN/m or less, further preferably 25 mN/m or less, still more preferably 20 mN/m or less, still more preferably 18 mN/m or less.

The oil-water interfacial tension of the organic compound can be measured by the method described in Examples.

The content of the primary alcohol in the organic compound is preferably 1% by mass or more, more preferably 2% by mass or more, further preferably 3% by mass or more, still more preferably 5% by mass or more, still more preferably 10% by mass or more, still more preferably 20% by mass or more, still more preferably 30% by mass or more, still more preferably 40% by mass or more, still more preferably 50% by mass or more from the viewpoint of satisfactorily exhibiting the delivery performance of the primary alcohol, and the viewpoint of improving the production efficiency, and then, is preferably 100% by mass or less from the viewpoint of increasing the encapsulation rate, and the viewpoint of improving the long-term retention.

When the primary alcohol contained in the organic compound is a fragrance component, and the organic compound contains the other components other than the primary alcohol, the content of the other component in the organic compound falls within a range where the effect of the present invention is not impaired, and is an amount based on the function exerted by such the other component. For example, when the organic compound is a compounded fragrance, the content of another component in the organic compound becomes an amount based on the desired scent. In the present invention, since the primary alcohol may be encapsulated in the silica capsule at a high encapsulation rate, a combination can be made without any limitation on the type or amount of the primary alcohol.

<Shell>

The shell of the silica capsule of the present invention contains silica as a constituent component.

The shell of the silica capsule of the present invention preferably contains silica, which is a hydrolyzed polycondensate of alkoxysilane, as a constituent component.

The shell of the silica capsule of the present invention is preferably formed and obtained by a sol-gel reaction using alkoxysilane as a precursor from the viewpoint of reducing the particle size of the silica capsule, the viewpoint of increasing the encapsulation rate, the viewpoint of improving the long-term retention, the viewpoint of exhibiting the delivery performance of the primary alcohol, and the viewpoint of improving the production efficiency.

The "sol-gel reaction" in the present invention means a reaction in which alkoxysilane forms silica that is a constituent component of the shell through a sol and gel state by hydrolysis and polycondensation reactions. Specifically, for example, in the reaction, tetraalkoxysilane is hydrolyzed, a silanol compound produces a siloxane oligomer through a dehydration condensation reaction and a dealcohol condensation reaction, and further a dehydration condensation reaction proceeds so as to form silica.

Further, the shell of the silica capsule of the present invention may contain an inorganic polymer other than silica, as a constituent component, within a range where the effect of the present invention is not impaired. The inorganic polymer in the present invention refers to a polymer containing an inorganic element. Examples of the inorganic polymer include a polymer composed of only an inorganic element, and a polymer that has a main chain composed of only an inorganic element and an organic group as a side chain or a substituent.

The inorganic polymer is preferably a metal oxide containing a metal element or a metalloid element, and is further preferably a polymer formed and obtained by using metal alkoxide $[M(OR)_x]$ as a precursor through a reaction similar to the above-described sol-gel reaction of silica. Here, M is a metal or metalloid element, and R is a hydrocarbon group.

Examples of the metal or metalloid element constituting metal alkoxide include titanium, zirconium, aluminum, and zinc.

The alkoxysilane is preferably tetraalkoxysilane from the viewpoint of reducing the particle size of the silica capsule, the viewpoint of increasing the encapsulation rate of the organic compound, the viewpoint of improving the long-term retention, the viewpoint of satisfactorily exhibiting the delivery performance of the primary alcohol, and the viewpoint of improving the production efficiency.

The tetraalkoxysilane preferably has an alkoxy group having 1 to 4 carbon atoms from the viewpoint of promoting a sol-gel reaction, is more preferably one or more selected from the group consisting of tetramethoxysilane, tetraethoxysilane, and tetraisopropoxysilane, further preferably one or more selected from the group consisting of tetramethoxysilane and tetraethoxysilane, still more preferably tetraethoxysilane.

[Method of producing silica microcapsules]

The shell of the silica capsule of the present invention is preferably produced by the following step I.

Step I: subjecting an emulsified liquid obtained by emulsifying an aqueous phase component containing a cationic surfactant, and an oil phase component containing a primary alcohol-containing organic compound and tetraalkoxysilane, to a sol-gel reaction under an acidic condition, thereby forming a silica capsule that has a core, and a shell whose constituent component is silica to obtain a water dispersion containing the silica capsule.

(Step I)

The step I is a step of subjecting an emulsified liquid obtained by emulsifying an aqueous phase component containing a cationic surfactant, and an oil phase component containing a primary alcohol-containing organic compound and tetraalkoxysilane, to a sol-gel reaction under an acidic condition, thereby forming a silica capsule that has a core, and a shell whose constituent component is silica, to obtain a water dispersion containing the silica capsule.

Examples of the cationic surfactant in the step I include alkylamine salts, and alkyl quaternary ammonium salts. The number of carbon atoms in the alkyl group of the alkylamine salt and the alkyl quaternary ammonium salt is preferably 10 or more, more preferably 12 or more, further preferably 14 or more, and is preferably 22 or less, more preferably 20 or less, further preferably 18 or less.

Examples of the alkylamine salt include alkylamine acetates such as laurylamine acetate, and stearylamine acetate.

Examples of the quaternary ammonium salt include an alkyltrimethyl ammonium salt, a dialkyldialkyl ammonium salt, and an alkylbenzyldimethyl ammonium salt.

Examples of the alkyltrimethyl ammonium salt include alkyltrimethyl ammoniumchlorides such as lauryltrimethyl ammoniumchloride, cetyltrimethyl ammoniumchloride, and stearyltrimethyl ammoniumchloride; and alkyltrimethyl ammoniumbromides such as lauryltrimethyl ammoniumbromide, cetyltrimethyl ammoniumbromide, and stearyltrimethyl ammoniumbromide.

Examples of the dialkyldimethylammonium salt include dialkyldimethyl ammoniumchlorides such as distearyldimethyl ammoniumchloride; and dialkyldimethyl ammoniumbromides such as distearyldimethyl ammoniumbromide.

Examples of the alkylbenzyldimethyl ammonium salt include alkylbenzyldimethyl ammoniumchloride, and alkylbenzyldimethyl ammoniumbromide.

Among these, the cationic surfactant is preferably a quaternary ammonium salt, more preferably an alkyltrimethyl ammonium salt having an alkyl group having 10 or more and 22 or less carbon atoms, further preferably alkyltrimethyl ammoniumchloride having an alkyl group having 10 or more and 22 or less carbon atoms, still more preferably one or more selected from the group consisting of lauryltrimethyl ammoniumchloride, stearyltrimethyl ammoniumchloride, and cetyltrimethyl ammoniumchloride, still more preferably cetyltrimethyl ammoniumchloride.

In the step I, in a range where the effect of the present invention is not impaired, besides the cationic surfactant, another emulsifier may be contained. Examples of the other emulsifier include a polymer dispersant, a nonionic surfactant, an anionic surfactant, and an amphoteric surfactant.

The content of the cationic surfactant in the aqueous phase component in the step I is preferably 0.1% by mass or more, more preferably 0.3% by mass or more, further preferably 0.4% by mass or more from the viewpoint of a dispersion stability of emulsified droplets, and is preferably 10% by mass or less, more preferably 5% by mass or less, further preferably 2% by mass or less, still more preferably 1% by mass or less, still more preferably 0.7% by mass or less from the viewpoint of suppressing emulsifier micelles from being formed by surplus emulsifier not contributing to the dispersion stability of the emulsified liquid and improving the encapsulation efficiency.

The amount of the oil phase component in the total amount of the emulsified liquid obtained in the step I is preferably 5% by mass or more, more preferably 10% by mass or more, further preferably 15% by mass or more from the viewpoint of production efficiency, and is preferably 50% by mass or less, more preferably 45% by mass or less, further preferably 40% by mass or less, still more preferably 35% by mass or less from the viewpoint of obtaining a stable emulsified liquid.

The amount of tetraalkoxysilane to be added in the step I is preferably 10% by mass or more, more preferably 12% by mass or more, further preferably 14% by mass or more, relative to the amount of the organic compound in the step I, from the viewpoint of promoting the sol-gel reaction, and forming a sufficiently dense shell, and is preferably 60% by mass or less, more preferably 50% by mass or less, further preferably 40% by mass or less, still more preferably 35% by mass or less, relative to the amount of the organic compound in the step I, from the viewpoint of suppressing surplus tetraalkoxysilane from remaining in the organic compound.

Here, the amount of tetraalkoxysilane to be added in the step I is the ratio when the amount of the organic compound in the step I is 100% by mass.

The total amount of the organic compound and tetraalkoxysilane relative to the total amount of the aqueous phase component and the oil phase component in the step I is preferably 5% by mass or more, more preferably 10% by mass or more, further preferably 15% by mass or more, and then is preferably 50% by mass or less, more preferably 45% by mass or less, further preferably 40% by mass or less, still more preferably 35% by mass or less from the viewpoint of obtaining a stable emulsified liquid.

The step I preferably includes the following steps 1-1 to 1-4.

Step 1-1: preparing an aqueous phase component containing a cationic surfactant.

Step 1-2: preparing an oil phase component by mixing an organic compound with tetraalkoxysilane.

Step 1-3: mixing and emulsifying the aqueous phase component obtained in the step 1-1 and the oil phase component obtained in the step 1-2 to obtain an emulsified liquid.

Step 1-4: subjecting the emulsified liquid obtained in the step 1-3 to a first sol-gel reaction step to form a silica capsule that has a core, and a shell whose constituent component is silica.

Although a stirring unit used for preparing the emulsified liquid is not particularly limited, a homogenizer having a strong shear force, a highpressure disperser, an ultrasonic disperser, etc. can be used. Further, a homomixer, "DISPER" (product name, manufactured by Primix Corporation), "CLEARMIX" (product name, manufactured by M Technique Co. Ltd.), "CAVITRON" (product name, manufactured by Pacific Machinery & Engineering Co. Ltd.), etc. can also be used.

From the viewpoint of production stability, the temperature during mixing and emulsification of the aqueous phase component and the oil phase component is preferably 5°C or more, more preferably 8°C or more, further preferably 10°C or more, still more preferably 15°C or more, and is preferably 50°C or less, more preferably 40°C or less, further preferably 35°C or less, still more preferably 30°C or less.

It is desirable that the rotation speed, etc. of the stirring unit and the mixing and emulsification time of the aqueous phase component and the oil phase component are appropriately adjusted so that the median diameter $D_{50}$ of emulsified droplets of the emulsified liquid falls within a range to be described below.

The median diameter $D_{50}$ of the emulsified droplets in the emulsified liquid in the step I is preferably 0.1 $\mu$m or more, more preferably 0.2 $\mu$m or more, further preferably 0.3 $\mu$m or more from the viewpoint of reducing the specific surface area with respect to the environment outside the silica capsule and increasing the long-term retention, and is preferably 50 $\mu$m or less, more preferably 30 $\mu$m or less, further preferably 10 $\mu$m or less, still more preferably 5 $\mu$m or less, still more preferably 3 $\mu$m or less, still more preferably 2 $\mu$m or less from the viewpoint of reducing the particle size of the silica capsule, and the viewpoint of the physical strength of the silica capsule.

The median diameter $D_{50}$ of the emulsified droplets can be measured by the method described in Examples.

The initial pH of the sol-gel reaction in the step I is preferably 3.0 or more, more preferably 3.3 or more, further preferably 3.5 or more from the viewpoint of maintaining the balance between a hydrolysis reaction and a condensation reaction of tetraalkoxysilane, and the viewpoint of suppressing the formation of highly hydrophilic sol and promoting the progress of encapsulation, and is preferably 4.5 or less, more preferably 4.3 or less, further preferably 4.1 or less from the viewpoint of suppressing co-occurrence of formation of a silica shell and aggregation of emulsified droplets and obtaining the silica capsule having a dense shell.

Any acidic or alkaline pH adjuster may be used from the viewpoint of adjustment to a desired initial pH according to the strength of acidity or alkalinity of the oil phase component containing the organic compound.

The pH of the emulsified liquid may also be a desired value or less. In such a case, it is desirable that adjustment is performed by using an alkaline pH adjuster to be described below.

That is, the step 1-4 may be preferably the following step 1-4'.

Step 1-4': adjusting the pH of the emulsified liquid obtained in the step 1-3 by using a pH adjuster, and carrying out the first sol-gel reaction step to form a silica capsule having a core and a shell, and to obtain a water dispersion containing the silica capsule.

Examples of the acidic pH adjuster include inorganic acids such as hydrochloric acid, nitric acid, and sulfuric acid, organic acids such as acetic acid, and citric acid, and solutions obtained by adding a cation exchange resin or the like to water, ethanol or the like. Hydrochloric acid, sulfuric acid, nitric acid, and citric acid are preferred.

Examples of the alkaline pH adjuster include sodium hydroxide, sodium hydrogen carbonate, potassium hydroxide, ammonium hydroxide, diethanolamine, triethanolamine, and trishydroxymethylaminomethane. Sodium hydroxide, and ammonium hydroxide are preferred.

As for the reaction temperature of the sol-gel reaction in the step I, any value may be selected as long as it is equal to or greater than a melting point of water contained as the aqueous phase and is equal to or smaller than a boiling point. However, it is desirable to set the temperature within a certain range from the viewpoint of controlling the balance between a hydrolysis reaction and a condensation reaction in the sol-gel reaction, and forming a dense shell. The range is preferably 5°C or more and 60°C or less, more preferably 10°C or more and 50°C or less, further preferably 15°C or more and 40°C or less.

The reaction time of the sol-gel reaction in the step I is preferably 0.5 h or more, more preferably 1 h or more, further preferably 5 h or more, still more preferably 10 h or more, and is preferably 50 h or less, more preferably 40 h or less, further preferably 30 h or less when a reaction start is defined as the point in time when the inside of the reaction system reaches a predetermined reaction temperature.

Further, regarding the shell of the silica capsule of the present invention, from the viewpoint of increasing the encapsulation rate of the organic compound, the viewpoint of improving the long-term retention, and the viewpoint of satisfactorily exhibiting the delivery performance of the primary alcohol, it is desirable that the shell has an inner shell that contains silica, which is a hydrolyzed polycondensate of alkoxysilane, as a constituent component, and further an outer shell that contains silica, which is a hydrolyzed polycondensate of alkoxysilane, as a constituent component, on the outside of the inner shell. In a specific example, it is desirable that such a silica capsule contains, as a constituent component, silica formed and obtained by performing two sol-gel reaction steps in which tetraalkoxysilane as a silica precursor is further added to the silica capsule (1) obtained in the step I (hereinafter, referred to as the silica capsule (1)), that is, the water dispersion containing the silica capsule (1). That is, in this case, the silica capsule of the present invention is preferably produced by a method including the following steps 1 and 2.

Step 1: subjecting an emulsified liquid obtained by emulsifying an aqueous phase component containing a cationic surfactant, and an oil phase component containing a primary alcohol-containing organic compound and tetraalkoxysilane, to a sol-gel reaction under an acidic condition to form a silica capsule (1) that has a core, and a first shell whose constituent component is silica, and to obtain a water dispersion containing the silica capsule (1).

Step 2: further adding tetraalkoxysilane to the silica capsule (1)-containing water dispersion obtained in the step 1 and performing a sol-gel reaction, thereby forming a silica capsule having a second shell that encloses the first shell.

Further, in the present specification, when the step 1 and the step 2 are performed, "enclosing a first shell" means enclosing the first shell of the silica capsule (1) formed in the step 1, and also includes enclosing the silica capsule (1).

Through the step 2, a shell is further formed on the silica capsule formed in the step 1. Thus, it is thought that the silica capsule obtained in the step 2 becomes a silica capsule having an increased shell thickness as a whole, and becomes a silica capsule having a shell in which an inner shell is the shell formed in the step 1, and an outer shell is the shell formed in the step 2.

Hereinafter, when the step 1 and the step 2 are performed, the first shell formed by the step 1 is also referred to as a "first shell," and the second shell formed by the step 2 is also referred to as a "second shell."

(step 2)

When the shell of the silica capsule of the present invention contains silica formed and obtained by performing two sol-gel reaction steps, as a constituent component, the production method of the silica capsule of the present invention includes a step 2 as well as the step 1.

The step 2 is a step of further adding tetraalkoxysilane to the silica capsule (1)-containing water dispersion obtained in the step 1 and performing a sol-gel reaction, thereby forming a silica capsule having a second shell that encloses the first shell.

The amount of tetraalkoxysilane to be added in the step 2 is preferably 7% by mass or more, more preferably 10% by mass or more, further preferably 15% by mass or more, relative to the amount of the organic compound in the step 1, from the viewpoint of forming the second shell that encloses the first shell, and is preferably 200% by mass or less, more preferably 170% by mass or less, further preferably 150% by mass or less, relative to the amount of the organic compound in the step 1, from the viewpoint of suppressing formation of silica sol dispersed in the aqueous phase, and improving the dispersion stability of the silica capsule.

Here, the amount of tetraalkoxysilane to be added in the step 2 is a ratio when the amount of the organic compound in the step 1 is 100% by mass.

In the present invention, when the entire amount of the water dispersion obtained in the step 1 is provided to the step 2, the amounts of the organic compound and tetraalkoxysilane in the step 1 are amounts of the organic compound and tetraalkoxysilane used in the step 1, respectively. When a part of the water dispersion obtained in the step 1 is provided to the step 2, the amounts of the organic compound and tetraalkoxysilane in the step 1 are amounts obtained through a scale conversion from amounts of the organic compound and tetraalkoxysilane used in the step 1, respectively, by using the amount of the water dispersion that is obtained in the step 1 and is provided to the step 2.

In the step 2, the entire amount of tetraalkoxysilane to be added to the silica capsule (1)-containing water dispersion obtained in the step 1 may be added at once, or intermittent addition by division, or continuous addition may be carried out. However, from the viewpoint of forming a highly dense second shell, it is desirable that addition is carried out through continuous dropping.

When tetraalkoxysilane is continuously added dropwise, the dropping time can be appropriately set according to the production scale, but is preferably 5 min or more, more preferably 10 min or more, further preferably 30 min or more, and is preferably 1,200 min or less, more preferably 1,000 min or less, further preferably 500 min or less from the viewpoint of suppressing separation of the added tetraalkoxysilane and the water dispersion.

In the present invention, the total addition amount of tetraalkoxysilane when the step 1 and the step 2 are included, that is, the total addition amount of tetraalkoxysilane used in the step 1 and the step 2, is preferably 30% by mass or more, more preferably 35% by mass or more, further preferably 40% by mass or more, and is preferably 250% by mass or less, more preferably 200% by mass or less, further preferably 150% by mass or less, relative to the amount of the organic compound in the step 1. When the total addition amount of tetraalkoxysilane falls within the above-described range, the encapsulated organic compound can be retained for a long period of time.

Here, the total addition amount of tetraalkoxysilane (the total addition amount of tetraalkoxysilane used in the step 1 and the step 2) is a ratio when the amount of the organic compound in the step 1 is 100% by mass.

In the present invention, the total amount of the organic compound and tetraalkoxysilane in the step 1 is preferably 20% by mass or less, more preferably 18% by mass or less, further preferably 15% by mass or less, still more preferably 10% by mass or less, still more preferably 7% by mass or less, relative to the total amount of the water dispersion before the addition of tetraalkoxysilane in the step 2, from the viewpoint of improving the long-term retention of the organic compound, and is preferably 2% by mass or more, more preferably 3% by mass or more, further preferably 5% by mass or more, relative to the total amount of the water dispersion before the addition of tetraalkoxysilane in the step 2, from the viewpoint of production efficiency.

In order to adjust the total amount of the organic compound and tetraalkoxysilane in the step 1 relative to the total amount of the water dispersion before the addition of tetraalkoxysilane in the step 2, the step 1 may be performed such that amounts of the organic compound and tetraalkoxysilane in the step 1 and the total amount of the water dispersion obtained in the step 1 fall within above ranges, or water may be further added to the water dispersion obtained in the step 1 so as to perform dilution.

In the present invention, from the viewpoint of production efficiency, in the step 2, before tetraalkoxysilane is added, the water dispersion obtained in the step 1 may be diluted with water. That is, the step 2 may be the following step 2'.

Step 2': diluting the silica capsule (1)-containing water dispersion obtained in the step 1 through addition of water, and then further adding tetraalkoxysilane and performing a sol-gel reaction, thereby forming a silica capsule having a second shell that encloses the first shell.

The total amount of the organic compound and tetraalkoxysilane in the step 1 is preferably 3% by mass or more, more preferably 5% by mass or more, further preferably 10% by mass or more, still more preferably 15% by mass or more, and is preferably 50% by mass or less, more preferably 40% by mass or less, further preferably 35% by mass or less, further preferably 30% by mass or less, relative to the total amount of the undiluted water dispersion obtained in the step 1.

Further, in the present invention, when the entire amount of the water dispersion obtained in the step 1 is provided to the step 2', the amounts of the organic compound and tetraalkoxysilane in the step 1 are amounts of the organic compound and tetraalkoxysilane used in the step 1. When a part of the water dispersion obtained in the step 1 is provided to the step 2', the amounts of the organic compound and tetraalkoxysilane in the step 1 are amounts obtained through a scale conversion from amounts of the organic compound and tetraalkoxysilane used in the step 1, by using the amount of the water dispersion that is obtained in the step 1 and is provided to the step 2.

The dilution ratio is preferably two times or more, more preferably 2.5 times or more, and is preferably 20 times or less, more preferably 10 times or less, more preferably 7 times or less.

In the present specification, the "dilution ratio" is a mass ratio of the total amount of the water dispersion after dilution with water (hereinafter, also referred to as the "diluted water dispersion") to the total amount of the undiluted water dispersion that is obtained in the step 1 and is provided to the step 2' (the total amount of the diluted water dispersion/ the total amount of the undiluted water dispersion that is obtained in the step 1 and is provided to the step 2').

The reaction temperature of the sol-gel reaction in the step 2 or the step 2' can be arbitrarily selected as long as it is equal to or greater than a melting point of water contained as a dispersion medium, and is equal to or smaller than a boiling point. However, it is preferably 5°C or more, more preferably 10°C or more, further preferably 15°C or more, and is preferably 60°C or less, more preferably 50°C or less, further preferably 40°C or less from the viewpoint of controlling the balance between a hydrolysis reaction and a condensation reaction in the sol-gel reaction, and forming a dense shell. The sol-gel reaction of the step 1 and the sol-gel reaction of the step 2 or the step 2' may be carried out at different reaction temperatures.

When tetraalkoxysilane is added dropwise in the step 2, it is desirable that the reaction is further continued after the addition is completed. The reaction time of the sol-gel reaction subsequent to the completion of addition is preferably 0.5 h or more, more preferably 1 h or more, further preferably 5 h or more, still more preferably 10 h or more, and is preferably 50 h or less, more preferably 40 h or less.

In the present invention, an organic polymer compound may be further added to the silica capsule-containing water dispersion obtained in the step I.

Further, in the present invention, when the step 1 and the step 2 are included, in the step 2 or the step 2', an organic polymer compound may be further added to the silica capsule (1)-containing water dispersion obtained in the step 1. Here, the organic polymer compound means a compound having a weight average molecular weight of 5,000 or more.

The organic polymer compound is preferably one or more selected from the group consisting of a cationic polymer and a nonionic polymer.

The nonionic polymer means a water-soluble polymer that does not have electric charges in water. By using the nonionic polymer, it is possible to impart a function based on the purpose of the silica capsule to the silica capsule.

In a case where the cationic polymer or the nonionic polymer is used as for the organic polymer compound, for example, when the silica capsule related to the present invention is used for a fiber treatment agent composition or the like such as a softener composition, an improvement in adsorptivity to fibers can be expected.

The "water soluble polymer" in the present specification refers to a polymer, in which when the polymer (that has reached a constant weight through drying at 105°C for 2 h) is dissolved in 100 g of water of 25°C, the amount of the dissolution is 1 mg or more.

Examples of the nonionic polymer include a polymer having a structural unit derived from a nonionic monomer, and a water-soluble polysaccharide (cellulose-based, gum-based, starch-based, etc.) and derivatives thereof.

Examples of the nonionic monomer include (meth)acrylate having an aliphatic alcohol-derived hydrocarbon group having 1 or more and 22 or less carbon atoms; styrene-based monomers such as styrene; aromatic-group containing (meth)acrylates such as benzyl(meth)acrylate; vinyl acetate; vinylpyrrolidone; vinyl alcohol; polyalkyleneglycol(meth)acrylates such as polyethyleneglycolmono(meth)acrylate; alkoxypolyalkyleneglycolmono(meth)acrylates such as methoxypolyethyleneglycolmono(meth)acrylate, and octoxypolyethyleneglycolmono(meth)acrylate; and (meth)acrylamide.

The nonionic polymer is preferably one or more selected from the group consisting of polyvinylpyrrolidone, a copolymer of vinylpyrrolidone and another nonionic monomer such as a vinylpyrrolidone/acetatevinyl copolymer, and cellulose-based polymers such as hydroxyethyl cellulose, hydroxypropyl cellulose, hydroxypropylmethyl cellulose, and hydroxyethylmethyl cellulose, and is more preferably one or more selected from the group consisting of polyvinylpyrrolidone and hydroxypropyl cellulose.

Examples of the cationic polymer include not only a polymer containing a quaternary ammonium salt group, but also a polymer having a nitrogen-based cationic group, and a polymer that can become cationic through pH adjustment. In the step 2 or the step 2', when the organic polymer compound is further added to the silica capsule (1)-containing water dispersion obtained in the step 1, the use of the cationic polymer can alleviate a situation where the silica capsules (1) obtained in the step 1 are likely to aggregate in the water dispersion, and thus, in the subsequent step 2 or step 2', formation of coarse particles or the like can be suppressed.

Examples of the cationic polymer include polydiallyldimethyl ammonium salts such as poly(diallyldimethyl ammoniumchloride), poly(acrylic acid-co-diallyldimethyl ammoniumchloride), poly(acrylamide-co-diallyldimethyl ammoniumchloride), and poly(acrylamide-co-acrylic acid-co-diallyldimethyl ammoniumchloride) and copolymers thereof, poly(2-(methacryloyloxy)ethyltrimethyl ammoniumchloride), polyethyleneimine, polyallylamine, cationized cellulose, cationized guar gum, cationized tara gum, cationized Fenugreek gum, and cationized locust bean gum. Among them, a polydiallyldimethyl ammonium salt and its copolymer are preferred, one or more selected from the group consisting of poly(diallyldimethyl ammoniumchloride), poly(acrylic acid-co-diallyldimethyl ammoniumchloride), and poly(acrylamide-co-acrylic acid-co-diallyldimethyl ammoniumchloride) is more preferred, and poly(diallyldimethyl ammoniumchloride) is further preferred.

The cationic group equivalent of the cationic polymer is preferably 1 meq/g or more, more preferably 3 meq/g or more, further preferably 4.5 meq/g or more, and is preferably 10 meq/g or less, more preferably 8 meq/g or less from the viewpoint of dispersibility of the silica capsule (1), the viewpoint of suppressing the formation of coarse particles, and the viewpoint of improving the long-term retention. The cationic polymer may contain an anionic group, but, in such a case, the anionic group equivalent contained in the cationic polymer is preferably 3.5 meq/g or less, more preferably 2 meq/g or less, further preferably 1 meq/g or less. In the present invention, as the cationic group equivalent of the cationic polymer, one calculated through calculation based on a monomer composition is used.

The addition amount of the organic polymer compound is preferably 0.05% by mass or more, more preferably 0.1% by mass or more, further preferably 0.2% by mass or more, and is preferably 5% by mass or less, more preferably 3% by mass or less, further preferably 2% by mass or less, relative to the amount of the water dispersion obtained in the step I or the step 1.

Here, the addition amount of the organic polymer compound is a ratio when the amount of the water dispersion obtained in the step I or the step 1 is 100% by mass.

The silica capsules of the present invention, which are obtained through the step I, and the step 2 or the step 2', are obtained in the form of dispersion in water. This may be used, as it is, depending on purposes, but in some cases, the silica capsules are separated for use. As for the separation method, a filtration method, a centrifugal separation method, etc. can be adopted.

(Silica microcapsule)

The silica capsule of the present invention is a silica capsule having a core that contains the organic compound, and a shell that encloses the core.

The shell of the silica capsule of the present invention encloses the core, contains silica as a constituent component, and preferably has an average thickness of 5 nm or more and 20 nm or less.

Further, the silica capsule of the present invention is preferably a silica capsule having a core that contains the organic compound, a first shell that encloses the core, and a second shell that encloses the first shell.

When the silica capsule of the present invention has the first shell and the second shell, the first shell encloses the core, contains silica as a constituent component, and preferably has an average thickness of 5 nm or more and 20 nm or less, and the second shell encloses the first shell, contains silica as a constituent component, and preferably has an average thickness of 10 nm or more and 100 nm or less.

The average thickness of the shells of the silica capsules, and the average thicknesses of the first shells and the second shells of the silica capsules can be measured by transmission electron microscope (TEM) observation. Specifically, under the observation of a transmission electron microscope, the thicknesses of the shells or the first shells and the second shells are actually measured on the photograph. This operation is performed by changing the field of view five times. From the obtained data, distributions of average thicknesses of the shells or the first shells and the second shells are obtained. The reference of magnification of the transmission electron microscope is 10,000 times to 100,000 times, but is appropriately adjusted according to the sizes of the silica capsules. Here, examples of the transmission electron microscope (TEM) include "JEM-2100" (product name, manufactured by JEOL Ltd.).

The median diameter $D_{50}$ of the silica capsules of the present invention is preferably 0.1 $\mu$m or more, more preferably 0.5 $\mu$m or more, further preferably 1 $\mu$m or more from the viewpoint of improving the long-term retention, and improving the dispersion stability of the silica capsule, and is preferably 100 $\mu$m or less, more preferably 50 $\mu$m or less, further preferably 30 $\mu$m or less, still more preferably 10 $\mu$m or less, still more preferably 7 $\mu$m or less from the viewpoint of improving the physical strength of the silica capsule, and improving the long-term retention.

The median diameter $D_{50}$ of the silica capsules can be measured by the method described in Examples.

The silica capsule of the present invention is formed by encapsulating a primary alcohol as the organic compound into the core at a high encapsulation rate. From this viewpoint, the encapsulation rate of the primary alcohol is preferably 30% or more, more preferably 40% or more, further preferably 50% or more, still more preferably 60% or more, still more preferably 70% or more, still more preferably 80% or more, still more preferably 90% or more, and is preferably 100% or less.

Further, since the silica capsule of the present invention contains the primary alcohol as the organic compound, when the organic compound is a compounded fragrance, multifarious compounded fragrances can be designed. From this viewpoint, the encapsulation rate of the primary alcohol is preferably 100% or less, more preferably 80% or less, further preferably 60% or less, still more preferably 40% or less, and is preferably 10% or more.
Specifically, the encapsulation rate of the primary alcohol is measured by the following method.

(Measurement of encapsulation rate)

Hexane containing dodecane and tridecane as internal standard substances at about 20 ppm (hereinafter, referred to as "reference A") is prepared, 0.62 g of ion-exchanged water and 20 mL of reference A are added to 0.04 g of the organic compound as an encapsulated component, and shaking is performed 10 times. Then, the upper layer passes through a membrane filter (for example, manufactured by Toyo Roshi Kaisha, Ltd., product name "DISMIC", model "13JP020AN"). Next, each component of the organic compound contained in this solution is measured by using gas chromatography, and the GC area value $\alpha$ (1) of each fragrance component per 1 mg/mL of the organic compound is obtained.

Apart from this, 20 mL of the reference A is added to a mixed solution (a total amount of 0.66 g) that is obtained by mixing silica capsules and water and contains 0.04 g of the organic compound as an encapsulated component, and then shaking is performed 10 times. Next, the upper layer passes through a membrane filter (for example, manufactured by Toyo Roshi Kaisha, Ltd., product name "DISMIC", model "13JP020AN"). Then, the organic compound contained in this solution is measured by using gas chromatography, and the GC area value $\beta$ (1) of each component of the organic compound per 1 mg/mL of the organic compound is obtained.

Then, the encapsulation rate of the primary alcohol contained in the organic compound is calculated according to the following formula (i).

$$\text{Encapsulation rate } (\%) = \{(\alpha(1) - \beta(1))/\alpha(1)\} \times 100 \, (\text{i})$$

The silica capsules of the present invention can be used for various purposes, and can be highly suitably used for, for example, various purposes, such as cosmetics, e.g., milky lotion, cosmetic liquid, cosmetic water, beauty serum, cream, gel formulation, hair treatment, and quasi-drugs, fiber treatment agents, e.g., a detergent, a softener, and an anti-wrinkle spray, sanitary products, e.g., paper diapers, and air fresheners.

The silica capsules of the present invention can be used by being blended with a composition such as a detergent composition, a fiber treatment agent composition, a cosmetic composition, an air freshener composition, and a deodorant composition. As the composition, a detergent composition such as a powder detergent composition and a liquid detergent composition, and a fiber treatment agent composition such as a softener composition are preferred; a fiber treatment agent composition is more preferred, and a softener composition is further preferred.

In relation to the above-described embodiment, the present invention further discloses the following silica microcapsules, a softener composition containing the silica microcapsules, and a method of producing the silica microcapsules.

<1> A silica microcapsule, which includes a shell, and a core containing one or more organic compounds inside the shell,

wherein the shell contains silica as a constituent component, and
the organic compound contains a primary alcohol.

<2> The silica microcapsule according to the above <1>, the content of the primary alcohol in the organic compound is preferably 1% by mass or more, more preferably 2% by mass or more, further preferably 3% by mass or more, still more preferably 5% by mass or more, still more preferably 10% by mass or more, still more preferably 20% by mass or more, still more preferably 30% by mass or more, still more preferably 40% by mass or more, still more preferably 50% by mass or more, and is preferably 100% by mass or less.

<3> The silica microcapsule according to the above <1> or <2>, the oil-water interfacial tension of the organic compound is preferably 3 mN/m or more, more preferably 4 mN/m or more, further preferably 5 mN/m or more, still more preferably 7 mN/m or more, and is preferably 40 mN/m or less, more preferably 30 mN/m or less, further preferably 25 mN/m or less, still more preferably 20 mN/m or less, still more preferably 18 mN/m or less.

<4> The silica microcapsule according to any one of the above <1> to <3>, the number of carbon atom of the primary alcohol is preferably 4 or more, more preferably 6 or more, further preferably 8 or more, and is preferably 18 or less, more preferably 16 or less, further preferably 14 or less, still more preferably 12 or less.

<5> The silica microcapsule according to any one of the above <1> to <4>, the cLogP of the primary alcohol is preferably 1.0 or more, more preferably 2.0 or more, further preferably 3.0 or more, and is preferably 7.0 or less, more preferably 6.5 or less, further preferably 6.0 or less, still more preferably 5.5 or less.

<6> The silica microcapsule according to any one of the above <1> to <5>, the primary alcohol is preferably one or more selected from the group consisting of fragrances, antibacterial agents, preservatives, repellents, and active pharmaceutical ingredients.

<7> The silica microcapsule according to any one of the above <1> to <5>, the primary alcohol is preferably a fragrance component.

<8> The silica microcapsule according to any one of the above <1> to <7>, the primary alcohol is preferably one or more selected from the group consisting of terpene-based primary alcohols, linear or branched aliphatic primary alcohols, and aromatic primary alcohols, more preferably one or more selected from the group consisting of terpene-based primary alcohols and aromatic primary alcohols.

<9> The silica microcapsule according to any one of the above <1> to <7>, the primary alcohol is one or more selected from the group consisting of geraniol, citronellol, nellol, 1-dodecanol, tetrahydrogeraniol, cis-3-hexenol, 2-phenylethylalcohol, 6-phenyl-1-hexanol, and benzyl alcohol.

<10> The silica microcapsule according to any one of the above <1> to <9>, the median diameter $D_{50}$ of the silica microcapsule is preferably 0.1 $\mu$m or more, more preferably 0.5 $\mu$m or more, further preferably 1 $\mu$m or more, and is preferably 100 $\mu$m or less, more preferably 50 $\mu$m or less, further preferably 30 $\mu$m or less, still more preferably 10 $\mu$m or less, still more preferably 7 $\mu$m or less.

<11> The silica microcapsule according to any one of the above <1> to <10>, the shell contains silica, which is a hydrolyzed polycondensate of alkoxysilane, as the constituent component.

<12> The silica microcapsule according to any one of the above <1> to <10>, the shell contains silica, which is formed and obtained by a sol-gel reaction using alkoxysilane as a precursor, as the constituent component.

<13> The silica microcapsule according to any one of the above <1> to <12>, the shell has an inner shell that contains silica, which is a hydrolyzed polycondensate of alkoxysilane, as a constituent component, and further an outer shell that contains silica, which is a hydrolyzed polycondensate of alkoxysilane, as a constituent component, on the outside of the inner shell.

<14> The silica microcapsule according to any one of the above <1> to <12>, the shell contains silica formed and obtained by performing two sol-gel reaction steps of alkoxysilane, as the constituent component.

<15> The silica microcapsule according to any one of the above <11> to <14>, the alkoxysilane is tetraethoxysilane.

<16> A softener composition containing the silica microcapsule according to any one of the above <1> to <15>.

<17> A method of producing a silica microcapsule, which includes a shell, and a core containing one or more organic compounds inside the shell,

wherein the shell contains silica as a constituent component,
the organic compound contains a primary alcohol, and
the following step I is included.
Step I: subjecting an emulsified liquid obtained by emulsifying an aqueous phase component containing a cationic surfactant and an oil phase component containing a primary alcohol-containing organic compound and tetraalkoxysilane, to a sol-gel reaction under an acidic condition, thereby forming a silica capsule that has a core, and a shell whose constituent component is silica, to obtain a water dispersion containing the silica capsule.

<18> The method of producing the silica microcapsule according to the above <17>, the median diameter $D_{50}$ of

the emulsified droplets in the emulsified liquid in the step I is preferably 0.1 μm or more, more preferably 0.2 μm or more, further preferably 0.3 μm or more, and is preferably 50 μm or less, more preferably 30 μm or less, further preferably 10 μm or less, still more preferably 5 μm or less, still more preferably 3 μm or less, still more preferably 2 μm or less,

<19> A method of producing a silica microcapsule, which includes a shell, and a core containing one or more organic compounds inside the shell,

wherein the shell contains silica as a constituent component,
the organic compound contains a primary alcohol, and
the following steps 1 and 2 are included.

Step 1: subjecting an emulsified liquid obtained by emulsifying an aqueous phase component containing a cationic surfactant, and an oil phase component containing an organic compound and tetraalkoxysilane, to a sol-gel reaction under an acidic condition to form a silica microcapsule (1) that has a core, and a first shell whose constituent component is silica, and to obtain a water dispersion containing the silica microcapsule (1).
Step 2: further adding tetraalkoxysilane to the silica microcapsule (1)-containing water dispersion obtained in the step 1, and performing a sol-gel reaction, thereby forming a silica microcapsule having a second shell that encloses the first shell.

<20> The method of producing the silica microcapsule according to the above <19>, the median diameter $D_{50}$ of the emulsified droplets in the emulsified liquid in the step 1 is preferably 0.1 μm or more, more preferably 0.2 μm or more, further preferably 0.3 μm or more, and is preferably 50 μm or less, more preferably 30 μm or less, further preferably 10 μm or less, still more preferably 5 μm or less, still more preferably 3 μm or less, still more preferably 2 μm or less.

<21> The method of producing the silica microcapsule according to the above <19> or <20>, the step 2 is the following step 2'.
Step 2': diluting the silica microcapsule (1)-containing water dispersion obtained in the step 1 through addition of water, and then further adding tetraalkoxysilane and performing a sol-gel reaction, thereby forming a silica microcapsule having a second shell that encloses the first shell.

<22> The method of producing the silica microcapsule according to the above <21>, in the step 2', the dilution ratio is preferably 2 times or more, more preferably 2.5 times or more, and is preferably 20 times or less, more preferably 10 times or less, more preferably 7 times or less.

<23> The method of producing the silica microcapsule according to any one of the above <19> to <22>, the step 1 includes the following steps 1-1 to 1-4.

Step 1-1: preparing an aqueous phase component containing a cationic surfactant.
Step 1-2: preparing an oil phase component by mixing an organic compound with tetraalkoxysilane.
Step 1-3: mixing and emulsifying the aqueous phase component obtained in the step 1-1 and the oil phase component obtained in the step 1-2 to obtain an emulsified liquid.
Step 1-4: subjecting the emulsified liquid obtained in the step 1-3 to a first sol-gel reaction step to form a silica microcapsule (1) that has a core, and a first shell whose constituent component is silica.

EXAMPLES

Various measurements in Examples and Comparative Examples were performed by the following methods.

(Median diameter $D_{50}$)

The median diameter $D_{50}$ of emulsified droplets and the median diameter $D_{50}$ of silica capsules were measured by using a laser diffraction/scattering particle diameter distribution measuring device "LA-960" (product name, manufactured by HORIBA, Ltd.). The measurement was performed using a flow cell, the medium was water, and the refractive index of a dispersed phase was set as 1.45-0i. An emulsified liquid or a water dispersion containing silica capsules was added into the flow cell, and the measurement was carried out at a concentration at which a transmittance of around 90% is exhibited so as to obtain the median diameter $D_{50}$ on a volume basis.

(Oil-water interfacial tension)

The oil-water interfacial tension of an organic compound encapsulated in a core was measured by a hanging drop method (pendant drop method). In a constant temperature room of 25°C, a contact angle meter "DropMaster series DM-501" (manufactured by Kyowa Interface Science Co. Ltd) was used. The analysis was performed through the Young-Laplace method by using software "FAMAS" (manufactured by Kyowa Interface Science Co. Ltd).

Example 1

(Step 1)

11.1 g of QUARTAMIN 60W (product name, manufactured by Kao corporation, cetyltrimethyl ammoniumchloride, effective content 30% by mass) was diluted with 188.89 g of ion-exchanged water to obtain an aqueous phase component. To this aqueous phase component, an oil phase component, which was prepared by mixing 24 g of a model fragrance 1 having blending ratios noted in Table 1 below (volume average cLogP value: 3.6, specific gravity: 0.88, oil-water interfacial tension: 11.9 mN/m) with 6 g of tetraethoxysilane (hereinafter, also referred to as "TEOS"), was added. The mixed solution was emulsified at room temperature (about 25 °C) by using a homomixer (manufactured by HsiangTai Machinery Industry Co., Ltd., model: HM-310, hereinafter, the same applies) under conditions of a rotation speed of 5,000 rpm for 10 min, and then a rotation speed of 5,200 rpm for 10 min to obtain an emulsified liquid. At this time, the median diameter $D_{50}$ of the emulsified droplets was 1.77 $\mu$m.

After the pH of the obtained emulsified liquid was adjusted to 3.8 by using a 0.1 N sodium hydroxide aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-1) that has a core made of the model fragrance 1 and a first shell made of silica.

(Step 2')

275 g of the water dispersion obtained in the step 1 was diluted through addition of 825 g of water (dilution ratio: 4 times). Then, while the obtained mixed solution was stirred at a liquid temperature of 30°C, 66 g of TEOS was added dropwise for 420 min. After the dropping, stirring was further continued for 17 h, and then the mixed solution was cooled so as to form a second shell that encloses the first shell. Then, a water dispersion containing a silica capsule (I) in which the model fragrance 1 was encapsulated in amorphous silica was obtained.

Table 1: Model fragrance 1

| Fragrance component name | Blending ratio (part by mass) | cLpgP |
|---|---|---|
| Hexyl butyrate | 4.0 | 3.8 |
| Rose oxide | 4.4 | 3.6 |
| I-Menthone | 4.2 | 2.9 |
| Linalool | 3.6 | 3.3 |
| Geraniol *1 | 21.1 | 3.3 |
| Citronellol *1 | 42.0 | 3.5 |
| Linalyl acetate | 17.0 | 4.4 |
| Others | 3.7 | 4.4 |
| *1: indicates primary alcohol | | |

Example 2

(Step 1)

1.87 g of QUARTAMIN 60W was diluted with 110.42 g of ion-exchanged water to obtain an aqueous phase component. To this aqueous phase component, an oil phase component, which was prepared by mixing 40 g of a model fragrance 2 having blending ratios noted in Table 2 below (volume average cLogP value: 3.9, specific gravity: 0.90, oil-water interfacial tension: 13.2 mN/m) with 6 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.78 $\mu$m.

After the pH of the obtained emulsified liquid was adjusted to 3.7 by using a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-2) that has a core made of the model fragrance 2 and a first shell made of silica.

(Step 2')

5.00 g of the water dispersion obtained in the step 1 was diluted through addition of 15.15 g of water (dilution ratio: 4 times). Then, while the obtained mixed solution was stirred at a liquid temperature of 30 °C, 1.18 g of TEOS was added. Stirring was continued for 24 h, and then the mixed solution was cooled so as to form a second shell that encloses the first shell. Then, a water dispersion containing a silica capsule (II) in which the model fragrance 2 was encapsulated in amorphous silica was obtained.

Table 2: Model fragrance 2

| Fragrance component name | Blending ratio (part by mass) | cLogP |
|---|---|---|
| 2-Phenylethyl alcohol *1 | 10 | 1.6 |
| Tetrahydrogeraniol *1 | 25 | 3.6 |
| Hexyl salicylate | 35 | 5.1 |
| Tetrahydrolinalool | 30 | 3.6 |
| *1: indicates primary alcohol | | |

Example 3

(Step 1)

1.87 g of QUARTAMIN 60W was diluted with 112.19 g of ion-exchanged water to obtain an aqueous phase component. To this aqueous phase component, an oil phase component, which was prepared by mixing 29.99 g of a model fragrance 3 having blending ratios noted in Table 3 below (volume average cLogP value: 4.3, specific gravity: 0.92, oil-water interfacial tension: 13.8 mN/m) with 7.46 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.78 $\mu$m.

After the pH of the obtained emulsified liquid was adjusted to 3.7 by using 0.17 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-3) that has a core made of the model fragrance 3 and a first shell made of silica.

(Step 2')

5.04 g of the water dispersion obtained in the step 1 was diluted through addition of 15.26 g of water (dilution ratio: 4 times). Then, while the obtained mixed solution was stirred at a liquid temperature of 30°C, 1.20 g of TEOS was added. Stirring was continued for 24 h, and then the mixed solution was cooled so as to form a second shell that encloses the first shell. Then, a water dispersion containing a silica capsule (III) in which the model fragrance 3 was encapsulated in amorphous silica was obtained. The median diameter $D_{50}$ of the silica capsules (III) was 1.68 $\mu$m.

Table 3: Model fragrance 3

| Fragrance component name | Blending ratio (part by mass) | cLogP |
|---|---|---|
| Hexyl salicylate | 30 | 5.1 |
| Iononea | 10 | 3.9 |
| Lilial | 20 | 4.4 |

(continued)

| Fragrance component name | Blending ratio (part by mass) | cLogP |
|---|---|---|
| Tetrahydrolinalool | 10 | 3.6 |
| Cis-3-hexenol *1 | 10 | 1.6 |
| Alcohol-C12 *1 | 20 | 5.1 |
| *1: indicates primary alcohol | | |

Example 4

(Step 1)

1.87 g of QUARTAMIN 60W was diluted with 110.88 g of ion-exchanged water to obtain an aqueous phase component. To this aqueous phase component, an oil phase component, which was prepared by mixing 30.00 g of a model fragrance 4 having blending ratios noted in Table 4 below (volume average cLogP value: 3.5, specific gravity: 0.85, oil-water interfacial tension: 12.1 mN/m) with 7.50 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.91 μm.
After the pH of the obtained emulsified liquid was adjusted to 3.8 by using 0.19 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-4) that has a core made of the model fragrance 4 and a first shell made of silica.

(Step 2')

4.99 g of the water dispersion obtained in the step 1 was diluted through addition of 14.98 g of water (dilution ratio: 4 times). Then, while the obtained mixed solution was stirred at a liquid temperature of 30°C, 1.19 g of TEOS was added. Stirring was continued for 24 h, and then the mixed solution was cooled so as to form a second shell that encloses the first shell. Then, a water dispersion containing a silica capsule (IV) in which the model fragrance 4 was encapsulated in amorphous silica was obtained. The median diameter $D_{50}$ of the silica capsules (IV) was 5.54 μm.

Table 4: Model fragrance 4

| Fragrance component name | Blending ratio (part by mass) | cLogP |
|---|---|---|
| Hexyl acetate | 20 | 2.8 |
| Citronellol *1 | 80 | 3.5 |
| *1: indicates primary alcohol | | |

Example 5

(Step 1)

1.66 g of QUARTAMIN 60W was diluted with 98.02 g of ion-exchanged water to obtain an aqueous phase component. To this aqueous phase component, an oil phase component, which was prepared by mixing 40.15 g of citronellol (cLogP value: 3.5, oil-water interfacial tension: 9.9 mN/m) with 10.00 g of TEOS, was added. The mixed solution was emulsified at room temperature (about 25°C) by using the homomixer under conditions of a rotation speed of 6,500 rpm for 5 min, and a rotation speed of 8,000 rpm for 3 min to obtain an emulsified liquid. At this time, the median diameter $D_{50}$ of the emulsified droplets was 2.12 μm.
After the pH of the obtained emulsified liquid was adjusted to 3.8 by using 0.46 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-5) that has a core made of citronellol and a first shell made of silica.

(Step 2')

24.96 g of the water dispersion obtained in the step 1 was diluted through addition of 74.93 g of water (dilution ratio: 4 times). Then, while the obtained mixed solution was stirred at a liquid temperature of 30°C, 6.00 g of TEOS was added dropwise for 420 min. After the dropping, stirring was further continued for 34 h, and then the mixed solution was cooled so as to form a second shell that encloses the first shell. Then, a water dispersion containing a silica capsule (V) in which citronellol was encapsulated in amorphous silica was obtained. The median diameter $D_{50}$ of the silica capsules (V) was 1.07 $\mu$m.

Example 6

(Step 1)

1.51 g of QUARTAMIN 60W was diluted with 88.53 g of ion-exchanged water to obtain an aqueous phase component. To this aqueous phase component, an oil phase component, which was prepared by mixing 24.01 g of a model fragrance 5 having blending ratios noted in Table 5 below (volume average cLogP value: 3.0, specific gravity: 0.95, oil-water interfacial tension: 5.7 mN/m) with 6.04 g of TEOS, was added. The mixed solution was emulsified at room temperature (about 25°C) by using the homomixer under conditions of a rotation speed of 6,500 rpm for 5 min, and a rotation speed of 8,000 rpm for 3 min to obtain an emulsified liquid. At this time, the median diameter $D_{50}$ of the emulsified droplets was 0.65 $\mu$m.
After the pH of the obtained emulsified liquid was adjusted to 3.3 by using 0.33 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-6) that has a core made of the model fragrance 5 and a first shell made of silica.
Although there are fragrance components whose cLogP values are not described in Table 5, as for the volume average cLogP value, the average value was obtained from fragrance components whose cLogP values are described. The same also applies to the following cases where there are fragrance components whose cLogP values are not described.

(Step 2')

24.96 g of the water dispersion obtained in the step 1 was diluted through addition of 74.93 g of water (dilution ratio: 4 times). Then, while the obtained mixed solution was stirred at a liquid temperature of 30°C, 6.00 g of TEOS was added dropwise for 420 min. After the dropping, stirring was further continued for 34 h, and then the mixed solution was cooled so as to form a second shell that encloses the first shell. Then, a water dispersion containing a silica capsule (VI) in which the model fragrance 5 was encapsulated in amorphous silica was obtained. The median diameter $D_{50}$ of the silica capsules (VI) was 5.70 $\mu$m.

Table 5: Model fragrance 5

| Fragrance component name | blending ratio (part by mass) | cLogP |
|---|---|---|
| CASSIS BASE Z.2908 | 5 | |
| Bergamot oil | 3 | |
| Benzyl alcohol *1 | 8 | 1.1 |
| Cis-3-hexenol *1 | 8 | 1.6 |
| 2-Phenylethyl alcohol *1 | 6 | 1.6 |
| Linalool | 10 | 3.3 |
| Geraniol *1 | 6 | 3.3 |
| Cis-jasmone | 18 | 3.6 |
| Linalool oxide | 1 | 2.0 |
| Nerolidol | 8 | 5.7 |
| Methyl dihydrojasmonate | 21 | 3.0 |

(continued)

| Fragrance component name | blending ratio (part by mass) | cLogP |
|---|---|---|
| Others | 6 | |
| *1: indicates primary alcohol | | |

Example 7

(Step 1)

0.75 g of QUARTAMIN 60W was diluted with 44.27 g of ion-exchanged water to obtain an aqueous phase component. To this aqueous phase component, an oil phase component, which was prepared by mixing 12.00 g of a model fragrance 6 having blending ratios noted in Table 6 below (volume average cLogP value: 3.9, specific gravity: 0.87, oil-water interfacial tension: 13.6 mN/m) with 3.00 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.27 $\mu$m.

After the pH of the obtained emulsified liquid was adjusted to 3.7 by using 0.58 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-7) that has a core made of the model fragrance 6 and a first shell made of silica.

(Step 2')

5.00 g of the water dispersion obtained in the step 1 was diluted through addition of 17.14 g of water (dilution ratio: 4 times). Then, while the obtained mixed solution was stirred at a liquid temperature of 30°C, 1.20 g of TEOS was added. Stirring was continued for 24 h, and then the mixed solution was cooled so as to form a second shell that encloses the first shell. Then, a water dispersion containing a silica capsule (VII) in which the model fragrance 6 was encapsulated in amorphous silica was obtained. the median diameter $D_{50}$ of the silica capsules (VII) was 1.06 $\mu$m.

Table 6: Model fragrance 6

| Fragrance component name | Blending ratio (part by mass) | cLogP |
|---|---|---|
| Cis-3-hexenol *1 | 0.60 | 1.6 |
| l-Menthone | 3.80 | 2.9 |
| Tetrahydrolinalool | 6.40 | 3.6 |
| Linalool | 12.10 | 3.3 |
| Geraniol *1 | 12.70 | 3.3 |
| Citronellol *1 | 25.40 | 3.5 |
| Camphor | 0.60 | 3.0 |
| Borneol | 1.10 | 2.9 |
| 1-Octen-3-yl acetate | 0.44 | 3.6 |
| Eucalyptus oil | 1.50 | 3.1 |
| Isobornyl acetate | 1.20 | 3.9 |
| Linalyl acetate | 18.10 | 4.4 |
| Isopropyl myristylate | 4.35 | 7.2 |
| Caryophyllene | 2.20 | 6.3 |
| Others | 9.51 | |
| *1: indicates primary alcohol | | |

Example 8

(Step 1)

1.54 g of QUARTAMIN 60W was diluted with 88.60 g of ion-exchanged water to obtain an aqueous phase component. To this aqueous phase component, an oil phase component, which was prepared by mixing 24.00 g of a model fragrance 7 having blending ratios noted in Table 7 below (specific gravity: 0.86, oil-water interfacial tension: 17.6 mN/m) with 6.06 g of TEOS, was added. The mixed solution was emulsified at room temperature (about 25 °C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 1.05 $\mu$m.

After the pH of the obtained emulsified liquid was adjusted to 3.7 by using 0.77 g of a 0.2 N hydrochloric acid aqueous solution, the liquid was transferred to a separable flask equipped with a stirring blade and a cooler. Then, while the liquid temperature was maintained at 30 °C, stirring was performed for 24 h to obtain a water dispersion containing a silica capsule (1-8) that has a core made of the model fragrance 7 and a first shell made of silica.

(Step 2')

101.10 g of the water dispersion obtained in the step 1 was diluted through addition of 304.65 g of water (dilution ratio: 4 times). Then, while the obtained mixed solution was stirred at a liquid temperature of 30 °C, 24.16 g of TEOS was added. Stirring was continued for 24 h, and then the mixed solution was cooled so as to form a second shell that encloses the first shell. Then, a water dispersion containing a silica capsule (VIII) in which the model fragrance 7 was encapsulated in amorphous silica was obtained.

Table 7: Model fragrance 7

| Fragrance component name | Blending ratio (part by mass) | cLogP |
|---|---|---|
| Lemon terpene | 30.0 | |
| Elemi oil | 5.0 | |
| Diphenylmethane | 1.0 | |
| Aldehyde C-10 | 0.7 | 3.8 |
| Terpinolene 20 | 2.0 | 4.9 |
| Citronellyl nitrile | 9.5 | 3.6 |
| Nerol *1 | 17.0 | 3.7 |
| Dihydromyrcenol | 2.0 | 3.5 |
| Mirac aldehyde | 0.7 | 4.7 |
| Isopropyl myristylate | 23.2 | 7.2 |
| Methyl dihydrojasmonate | 3.0 | 3.0 |
| Amyl cinnamic aldehyde | 4.0 | 4.3 |
| Others | 1.9 | |
| *1: indicates primary alcohol | | |

Example 9

(Step 1)

1.49 g of QUARTAMIN 60W was diluted with 88.52 g of ion-exchanged water to obtain an aqueous phase component. To this aqueous phase component, an oil phase component, which was prepared by mixing 24.13 g of a model fragrance 8 having blending ratios noted in Table 8 below (volume average cLogP value: 4.3, specific gravity: 0.88, oil-water interfacial tension: 16.3 mN/m) with 6.01 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the

emulsified droplets was 1.09 μm.

After the pH of the obtained emulsified liquid was adjusted to 3.7 by using 0.54 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-9) that has a core made of the model fragrance 8 and a first shell made of silica.

(Step 2')

100.22 g of the water dispersion obtained in the step 1 was diluted through addition of 305.58 g of water (dilution ratio: 4 times). Then, while the obtained mixed solution was stirred at a liquid temperature of 30°C, 24.00 g of TEOS was added. Stirring was continued for 24 h, and then the mixed solution was cooled so as to form a second shell that encloses the first shell. Then, a water dispersion containing a silica capsule (IX) in which the model fragrance 8 was encapsulated in amorphous silica was obtained.

Table 8: Model fragrance 8

| Fragrance component name | Blending ratio (part by mass) | cLogP |
|---|---|---|
| Orange guinea | 5.0 | |
| Octyl acetate | 5.0 | 3.8 |
| Limonene | 57.0 | 4.9 |
| α-Terpinene | 1.5 | 4.8 |
| Allyl cyclohexyl glycolate | 0.5 | 2.7 |
| 6-phenyl-1-hexanol *1 | 12.5 | 3.5 |
| Frutate | 3.0 | 3.6 |
| Fluoropearl | 3.0 | 3.1 |
| Methyl dihydrojasmonate | 12.5 | 3.0 |
| *1: indicates primary alcohol | | |

Example 10

(Step 1)

1.51 g of QUARTAMIN 60W was diluted with 88.47 g of ion-exchanged water to obtain an aqueous phase component. To this aqueous phase component, an oil phase component, which was prepared by mixing 23.95 g of a model fragrance 9 having blending ratios noted in Table 9 below (volume average cLogP value: 3.5, specific gravity: 0.95, oil-water interfacial tension: 8.0 mN/m) with 6.05 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.48 μm.

After the pH of the obtained emulsified liquid was adjusted to 3.7 by using 0.22 g of a 0.1 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-10) that has a core made of the model fragrance 9 and a first shell made of silica.

(Step 2')

99.88 g of the water dispersion obtained in the step 1 was diluted through addition of 300.84 g of water (dilution ratio: 4 times). Then, while the obtained mixed solution was stirred at a liquid temperature of 30°C, 24.00 g of TEOS was added. Stirring was continued for 24 h, and then the mixed solution was cooled so as to form a second shell that encloses the first shell. Then, a water dispersion containing a silica capsule (X) in which the model fragrance 9 was encapsulated in amorphous silica was obtained.

Table 9: Model fragrance 9

| Fragrance component name | Blending ratio (part by mass) | cLogP |
|---|---|---|
| Cis-3-hexenol *1 | 0.3 | 1.6 |
| 2-Phenylethyl alcohol *1 | 30.0 | 1.6 |
| Terpineol | 6.0 | 3.3 |
| Triplal | 3.0 | 2.9 |
| Citronellol *1 | 3.0 | 3.5 |
| Florosa | 7.0 | 2.0 |
| Eugenol | 1.5 | 2.7 |
| EthylLinalool | 6.0 | 3.9 |
| Tricyclodecenyl acetate | 1.2 | 2.9 |
| SANDALMYSORE CORE | 1.2 | 4.7 |
| Styralyl acetate | 1.2 | 2.5 |
| Jasmopyran Forte | 1.5 | 3.3 |
| Isopropyl myristylate | 12.1 | 7.2 |
| Amber core | 6.0 | 4.1 |
| Ethylene brassylate | 11.0 | 4.7 |
| Hexyl cinnamic aldehyde | 9.0 | 4.8 |
| *1: indicates primary alcohol | | |

Example 11

(Step 1)

1.50 g of QUARTAMIN 60W was diluted with 88.50 g of ion-exchanged water to obtain an aqueous phase component. To this aqueous phase component, an oil phase component, which was prepared by mixing 24.00 g of a model fragrance 10 having blending ratios noted in Table 10 below (volume average cLogP value: 3.3, specific gravity: 0.96, oil-water interfacial tension: 12.1 mN/m) with 6.04 g of TEOS, was added. The mixed solution was emulsified at room temperature (about 25°C) by using the homomixer under conditions of a rotation speed of 6,500 rpm for 5 min, and a rotation speed of 8,000 rpm for 5 min to obtain an emulsified liquid. At this time, the median diameter $D_{50}$ of the emulsified droplets was 0.86 $\mu$m.

After the pH of the obtained emulsified liquid was adjusted to 3.8 by using 0.52 g of a 0.1 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-11) that has a core made of the model fragrance 10 and a first shell made of silica.

(Step 2')

100.39 g of the water dispersion obtained in the step 1 was diluted through addition of 306.51 g of water (dilution ratio: 4 times). Then, while the obtained mixed solution was stirred at a liquid temperature of 30°C, 24.15 g of TEOS was added. Stirring was continued for 24 h, and then the mixed solution was cooled so as to obtain a water dispersion containing a silica capsule (XI) in which the model fragrance 10 was encapsulated in amorphous silica.

Table 10: Model fragrance 10

| Fragrance component name | Blending ratio (part by mass) | cLogP |
|---|---|---|
| Acetyl cedrene core T | 8.0 | 5.0 |
| Traseolide 100 | 7.5 | |

(continued)

| Fragrance component name | Blending ratio (part by mass) | cLogP |
|---|---|---|
| Isolongifolanone | 3.0 | 3.8 |
| Coumarin | 0.5 | 1.5 |
| 2-Phenylethyl alcohol *1 | 10.0 | 1.6 |
| Linalool | 10.0 | 3.3 |
| Benzyl acetate | 4.0 | 2.1 |
| Benzyl salicylate | 10.0 | 4.3 |
| Citronellol *1 | 10.0 | 3.5 |
| Dihydromyrcenol | 10.0 | 3.5 |
| Styralyl acetate | 1.0 | 2.5 |
| Jasmopyran Forte | 10.0 | 3.3 |
| Methyl dihydrojasmonate | 5.0 | 3.0 |
| Methyl ionone G | 3.0 | 4.8 |
| Hexyl cinnamic aldehyde | 8.0 | 4.8 |
| *1: indicates primary alcohol | | |

Example 12

(Step 1)

4.18 g of QUARTAMIN 60W was diluted with 1045.50 g of ion-exchanged water to obtain an aqueous phase component. To this aqueous phase component, an oil phase component, which was prepared by mixing 280.00 g of a model fragrance 11 having blending ratios noted in Table 11 below (volume average cLogP value: 3.7, specific gravity: 0.88, oil-water interfacial tension: 13.9 mN/m) with 70.10 g of TEOS, was added. The mixed solution was emulsified at room temperature (about 25°C) by using the homomixer under conditions of a rotation speed of 6,500 rpm for 5 min, and a rotation speed of 8,000 rpm for 17 min to obtain an emulsified liquid. At this time, the median diameter $D_{50}$ of the emulsified droplets was 1.16 $\mu$m.

After the pH of the obtained emulsified liquid was adjusted to 3.7 by using a 0.1 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-12) that has a core made of the model fragrance 11 and a first shell made of silica.

(Step 2)

To 1400 g of the water dispersion obtained in the step 1, 42.00 g of TEOS was added while stirring was performed at a liquid temperature of 30°C. Stirring was continued for 24 h, and then the mixed solution was cooled so as to obtain a water dispersion containing a silica capsule (XII) in which the model fragrance 11 was encapsulated in amorphous silica.

Table 11: Model fragrance 11

| Fragrance component name | Blending ratio (part by mass) | cLogP |
|---|---|---|
| Linalool | 22 | 3.3 |
| Linalyl acetate | 16 | 4.4 |
| Tetrahydrolinalool | 16 | 3.6 |
| Caryophyllene | 5 | 6.3 |
| Coumarin | 4 | 1.5 |

(continued)

| Fragrance component name | Blending ratio (part by mass) | cLogP |
|---|---|---|
| Eucalyptus oil | 3 | 3.1 |
| Isobornyl acetate | 3 | 3.9 |
| Ocimene | 3 | 4.8 |
| Borneol | 3 | 2.9 |
| Neryl acetate | 2 | 4.5 |
| Alpha pinene | 2 | 4.3 |
| Cis-3-hexenol *1 | 2 | 1.6 |
| Others | 19 | |
| *1: indicates primary alcohol | | |

Comparative Example 1

15% by mass of a Gohsenol GH-20 (product name, manufactured by The Nippon Synthetic Chemical Industry Co. Ltd., polyvinyl alcohol) aqueous solution was prepared.

27.31 g of a 15% by mass Gohsenol GH-20 aqueous solution was diluted with 175.67 g of ion-exchanged water to obtain an aqueous phase component. To this aqueous phase component, an oil phase component, which was prepared by mixing 84.48 g of the model fragrance 1, 6.61 g of methacrylic acid (manufactured by FUJIFILM Wako Pure Chemical Corporation), 4.76 g of NKESTER 1G (product name, manufactured by SHIN-NAKAMURA CHEMICAL CO. LTD., ethylene glycol dimethacrylate), and 0.18 g of V-65 (product name, manufactured by FUJIFILM Wako Pure Chemical Corporation, 2,2'-azobis(2,4-dimethylvaleronitrile)), was added. The mixed solution was emulsified at room temperature (about 25°C) by using the homomixer under conditions of a rotation speed of 6,500 rpm for 5 min, and then a rotation speed of 8,000 rpm for 5 min to obtain an emulsified liquid. At this time, the median diameter $D_{50}$ of the emulsified droplets was 2.31 $\mu$m.

The emulsified liquid was subjected to nitrogen replacement, and was stirred at 65°C for 4 h, and at 75°C for 3 h to obtain a suspension. The monomer unreaction rate was measured by using liquid chromatography, and as a result, methacrylic acid was 36.9%, and ethylene glycol dimethacrylate was 91.7%.

The suspension of Comparative Example 1 was observed by a microscope, and as a result, no core-shell type capsule was formed.

Comparative Example 2

0.005 g of cetyltrimethyl bromide was dissolved in 37.53 g of ion-exchanged water, and then 12.03 g of Ludox HS-40 (product name, manufactured by Dupont, colloidal silica, average particle size 12 nm) was added thereto. Through mixing, a dispersion was obtained. While the dispersion was stirred by using the homomixer at room temperature (about 25°C), 37.56 g of the model fragrance 10 was added thereto and mixing was performed. Immediately after the mixing, a milky white liquid was obtained, but when the liquid was left for about 5 min, an oil phase and an aqueous phase were separated.

Comparative Example 3

(Step 1)

1.67 g of QUARTAMIN 60W was diluted with 98.34 g of ion-exchanged water to obtain an aqueous phase component. To this aqueous phase component, an oil phase component, which was prepared by mixing 40.00 g of a model fragrance 12 (volume average cLogP value: 4.2, specific gravity: 0.95, oil-water interfacial tension: 21.0 mN/m) having blending ratios noted in Table 12 below and containing no primary alcohol with 3.00 g of TEOS, was added. The mixed solution was emulsified at room temperature (about 25°C) by using the homomixer to obtain an emulsified liquid. At this time, the median diameter $D_{50}$ of the emulsified droplets was 1.16 $\mu$m.

After the pH of the obtained emulsified liquid was adjusted to 3.8 by using a 0.1 N sodium hydroxide aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-C3) that has a core made of the model fragrance 12 and a first shell made of silica.

(Step 2')

25.00 g of the water dispersion obtained in the step 1 was diluted through addition of 75.00 g of water (dilution ratio 4 times). Then, while the obtained mixed solution was stirred at a liquid temperature of 30°C, 6.00 g of TEOS was added. Stirring was continued for 24 h, and then the mixed solution was cooled so as to form a second shell that encloses the first shell. Then, a water dispersion containing a silica capsule (C3) in which the model fragrance 12 was encapsulated in amorphous silica was obtained. The median diameter $D_{50}$ of the silica capsules (C3) was 2.18 $\mu$m.

Table 12: Model fragrance 12

| Fragrance component name | Blending ratio (part by mass) | cLogP |
|---|---|---|
| Iononea | 10 | 3.9 |
| Methyl dihydrojasmonate | 10 | 3.0 |
| Hexyl salicylate | 20 | 5.1 |
| Lilial | 20 | 4.4 |
| Tetrahydrolinalool | 10 | 3.6 |
| Hexyl cinnamic aldehyde | 20 | 4.8 |
| Hexyl acetate | 10 | 2.8 |

[Evaluation of silica capsules]

(Measurement method of encapsulation rate of fragrance component)

The encapsulation rate of each of the fragrance components of silica capsules (I) to (XII) obtained in Examples 1 to 12 was measured by the following method.
The same operation was also performed on Comparative Example 2 to measure the encapsulation rates of the fragrance components.
Hexane (reference A) containing dodecane and tridecane as internal standard substances at about 20 ppm was prepared. 0.62 g of ion-exchanged water and 20 mL of reference A were added to 0.04 g of each model fragrance, and shaking was performed 10 times. Then, the upper layer passed through a membrane filter (manufactured by Toyo Roshi Kaisha, Ltd., product name "DISMIC", model "13JP020AN"). Next, each fragrance component contained in this solution was measured by using gas chromatography, and the GC area value $\alpha$ (1) of each fragrance component per 1 mg/mL of the fragrance was obtained.
Apart from this, 20 mL of the reference A was added to a mixed solution (a total amount of 0.66 g) that was obtained by mixing the silica capsule-containing water dispersion obtained in Example or the liquid obtained in Comparative Example 2 with water and contained about 0.04 g of the model fragrance. Then, shaking was performed 10 times. Next, the upper layer passed through the membrane filter. Then, each fragrance component contained in this solution was measured by using gas chromatography to obtain the GC area value $\beta$ (1) of each fragrance component per 1 mg/mL of the fragrance contained in each water dispersion or the liquid obtained in Comparative Example 2.
The encapsulation rate of the fragrance component was calculated according to the following formula (i). The results are noted in Tables 14 to 26 below. In these Tables, the blank in the encapsulation rate indicates that no measurement was performed.

$$\text{encapsulation rate}(\%) = \{(\alpha(1) - \beta(1))/\alpha(1)\} \times 100 \quad \text{(i)}$$

(Evaluation method of long-term retention of fragrance component)

(1) Preparation of softener for evaluation

Each of silica capsule-containing water dispersions obtained in Examples 1, 3, 7, 9, 11, and 12 and the liquid obtained in Comparative Example 2 were added to a softener base having a composition noted in Table 13 below. Then, a softener for evaluation was prepared. The content of the encapsulated fragrance was set as 0.5% by mass in the softener for evaluation.

Table 13

| Softener base(*1) | Blending amount (part by mass) |
|---|---|
| Cationic softening base (*2) | 12 |
| Polyoxyethylene (40) lauryl ether | 3.5 |
| Calcium chloride | 0.2 |
| Ethylene glycol | 1.6 |
| Proxel BDN (*3) | 0.01 |
| Methylglycine trisodium diacetate | 0.01 |
| Ion-exchanged water | 82.68 |
| Total | 100 |

Each notation in Table 13 is as follows.

*1: Blending was performed such that the pH of the softener base became 3.2.
*2: Ester amine obtained by reacting plant fatty acid with triethanolamine at 1.65/1 mol was quaternarized with dimethyl sulfate by using a conventionally known method.
*3: Manufactured by Lonza Japan Co. Ltd.

(2) Evaluation of long-term retention of fragrance component

Each evaluation softener prepared in the above (1) was individually placed in a screw pipe and sealed, and was stored at 40°C.

After three weeks passed since the start of storage (after one week passed only when the silica capsule-containing water dispersions obtained in Example 7 and Example 11 were used), the screw pipe was taken out. 100 mg of the softener was scooped up with a pipette and was diluted with 10 g of ion-exchanged water, and then passed through a membrane filter (manufactured by Millipore Corporation, product name "Omnipore", product number "JAWP04700") so that capsules were collected on the membrane filter.

Further, the silica capsules were washed with 10 mL of ion-exchanged water, and then 10 mL of hexane on the membrane filter. Then, the silica capsules were immersed in 2 mL of acetonitrile containing tridecane as an internal standard at a concentration of 10 $\mu$g/mL, and were subjected to irradiation with ultrasonic waves for 60 min by using an ultrasonic irradiation device (manufactured by Branson, model "5510") under conditions of output power of 180 W, and oscillation frequency of 42 kHz so as to elute the fragrance within the silica capsules. This solution passed through a membrane filter (manufactured by Toyo Roshi Kaisha, Ltd., product name "DISMIC", model "13JP020AN") again. Then, each fragrance component contained in this solution was measured by using gas chromatography, and was set as the GC area value $\alpha$(2) of the fragrance component encapsulated in the silica capsules. Next, the fragrance retention rate of each fragrance component was measured according to the following formula (ii). The results are noted in Tables 14, 16, 20, 22, 24, 25, and 26 below. The blank in these Tables indicates that no measurement was performed.

Fragrance retention rate (%)={(GC area value α (2) of fragrance component encapsulated in silica capsules contained in 100 mg of softener after storage)/(GC area value ß (2) of fragrance component contained in 100 mg of softener)}×100　　　(ii)

The GC area value $\beta$ (2) of the fragrance component in the above formula was calculated from the composition of the model fragrance, the encapsulation rate of the fragrance, and the blending amount of silica capsules used for preparing the softener.

Table 14: Model fragrance 1 (Example 1)

| Fragrance component name | Blending ratio (part by mass) | Evaluation | |
| --- | --- | --- | --- |
| | | Encapsulation rate (%) | Fragrance retention rate (%) |
| Hexyl butyrate | 4.0 | | 57 |
| Rose oxide | 4.4 | | 51 |
| I-Menthone | 4.2 | 95 | 60 |
| Linalool | 3.6 | 89 | 46 |
| Geraniol *1 | 21.1 | 99 | 74 |
| Citronellol *1 | 42.0 | 98 | 66 |
| Linalyl acetate | 17.0 | 99 | 55 |
| Others | 3.7 | - | - |
| *1: indicates primary alcohol | | | |

Table 15: Model fragrance 2 (Example 2)

| Fragrance component name | Blending ratio (part by mass) | Evaluation |
| --- | --- | --- |
| | | Encapsulation rate (%) |
| 2-Phenylethyl alcohol *1 | 10 | 69 |
| Tetrahydrogeraniol *1 | 25 | 92 |
| Hexyl salicylate | 35 | 100 |
| Tetrahydrolinalool | 30 | 86 |
| *1: indicates primary alcohol | | |

Table 16: Model fragrance 3 (Example 3)

| Fragrance component name | Blending ratio (part by mass) | Evaluation | |
| --- | --- | --- | --- |
| | | Encapsulation rate (%) | Fragrance retention rate (%) |
| Hexyl salicylate | 30 | 100 | 60 |
| Iononea | 10 | 96 | 36 |
| Lilial | 20 | 97 | 34 |
| Tetrahydrolinalool | 10 | 91 | 23 |
| Cis-3-hexenol *1 | 10 | 42 | 0 |
| Alcohol-C12 *1 | 20 | 100 | 20 |
| *1: indicates primary alcohol | | | |

Table 17: Model fragrance 4 (Example 4)

| Fragrance component name | Blending ratio (part by mass) | Evaluation |
| --- | --- | --- |
| | | Encapsulation rate (%) |
| Hexyl acetate | 20 | 94 |

(continued)

| Fragrance component name | Blending ratio (part by mass) | Evaluation |
|---|---|---|
| | | Encapsulation rate (%) |
| Citronellol *1 | 80 | 94 |
| *1: indicates primary alcohol | | |

Table 18: Citronel (Example 5)

| Fragrance component name | Blending ratio (part by mass) | Evaluation |
|---|---|---|
| | | Encapsulation rate (%) |
| Citronellol *1 | 100 | 95 |
| *1: indicates primary alcohol | | |

Table 19: Model Fragrance 5 (Example 6)

| Fragrance component name | Blending ratio (part by mass) | Evaluation |
|---|---|---|
| | | Encapsulation rate (%) |
| CASSIS BASE Z.2908 | 5 | |
| Bergamot oil | 3 | |
| Benzyl alcohol *1 | 8 | 31 |
| Cis-3-hexenol *1 | 8 | 38 |
| 2-Phenylethyl alcohol *1 | 6 | 80 |
| Linalool | 10 | |
| Geraniol *1 | 6 | 88 |
| Cis-jasmone | 18 | 94 |
| Linalooloxide | 1 | |
| Nerolidol | 8 | 94 |
| Methyl dihydrojasmonate | 21 | 100 |
| Others | 6 | - |
| *1: indicates primary alcohol | | |

Table 20: Model Fragrance 6 (Example 7)

| Fragrance component name | Blending ratio (part by mass) | Evaluation | |
|---|---|---|---|
| | | Encapsulation rate (%) | Fragrance retention rate (%) *2 |
| Cis-3-hexenol *1 | 0.60 | 62 | 0 |
| l-Menthone | 3.80 | 95 | 66 |
| Tetrahydrolinalool | 6.40 | 100 | 57 |
| Linalool | 12.10 | 100 | 70 |
| Geraniol *1 | 12.70 | 95 | 100 |
| Citronellol *1 | 25.40 | 90 | 71 |
| Camphor | 0.60 | 87 | 74 |

(continued)

| Fragrance component name | Blending ratio (part by mass) | Evaluation | |
|---|---|---|---|
| | | Encapsulation rate (%) | Fragrance retention rate (%) *2 |
| Borneol | 1.10 | 99 | 65 |
| 1-Octen-3-yl acetate | 0.44 | 94 | 65 |
| Eucalyptus oil | 1.50 | | |
| Isobornyl acetate | 1.20 | 99 | 72 |
| Linalyl acetate | 18.10 | 93 | 77 |
| Isopropyl myristylate | 4.35 | | |
| Caryophyllene | 2.20 | 100 | 74 |
| Others | 9.51 | - | - |
| *1: indicates primary alcohol | | | |
| *2: indicates a fragrance retention rate one week after start of storage. | | | |

Table 21: Model Fragrance 7 (Example 8)

| Fragrance component name | Blending ratio (part by mass) | Evaluation |
|---|---|---|
| | | Encapsulation rate (%) |
| Lemon terpene | 30.0 | |
| Elemi oil | 5.0 | |
| Diphenylmethane | 1.0 | 100 |
| Aldehyde C-10 | 0.7 | |
| Terpinolene 20 | 2.0 | |
| Citronellyl nitrile | 9.5 | |
| Nerol *1 | 17.0 | 100 |
| Dihydromyrcenol | 2.0 | 95 |
| Mirac aldehyde | 0.7 | |
| Isopropyl myristylate | 23.2 | 100 |
| Methyl dihydrojasmonate | 3.0 | 100 |
| Amyl cinnamic aldehyde | 4.0 | 100 |
| Others | 1.9 | - |
| *1: indicates primary alcohol | | |

Table 22: Model Fragrance 8 (Example 9)

| Fragrance component name | Blending ratio (part by mass) | Evaluation | |
|---|---|---|---|
| | | Encapsulation rate (%) | Fragrance retention rate (%) |
| Orange guinea | 5.0 | | |
| Octyl acetate | 5.0 | 100 | |
| Limonene | 57.0 | 100 | 59 |
| $\alpha$-Terpinene | 1.5 | 100 | 32 |

(continued)

| Fragrance component name | Blending ratio (part by mass) | Evaluation | |
|---|---|---|---|
| | | Encapsulation rate (%) | Fragrance retention rate (%) |
| Allyl cyclohexyl glycolate | 0.5 | | |
| 6-phenyl-1-hexanol *1 | 12.5 | 95 | 100 |
| Frutate | 3.0 | 100 | 82 |
| Fluoropearl | 3.0 | | |
| Methyl dihydrojasmonate | 12.5 | 98 | 100 |
| *1: indicates primary alcohol | | | |

Table 23: Model Fragrance 9 (Example 10)

| Fragrance component name | Blending ratio (part by mass) | Evaluation |
|---|---|---|
| | | Encapsulation rate (%) |
| Cis-3-hexenol *1 | 0.3 | peak not detectable |
| 2-Phenylethyl alcohol *1 | 30.0 | 100 |
| Terpineol | 6.0 | 38 |
| Triplal | 3.0 | 82 |
| Citronellol *1 | 3.0 | 90 |
| Florosa | 7.0 | 72 |
| Eugenol | 1.5 | 88 |
| Ethyllinalool | 6.0 | 90 |
| Tricyclodecenyl acetate | 1.2 | |
| SANDALMYSORE CORE | 1.2 | 95 |
| Styralyl acetate | 1.2 | |
| Jasmopyran Forte | 1.5 | |
| Isopropyl myristylate | 12.1 | 100 |
| Amber core | 6.0 | |
| Ethylene brassylate | 11.0 | 98 |
| Hexylcinnamic aldehyde | 9.0 | 100 |
| *1: indicates primary alcohol | | |

Table 24: Model Fragrance 10 (Example 11)

| Fragrance component name | Blending ratio (part by mass) | Evaluation | |
|---|---|---|---|
| | | Encapsulation rate (%) | Fragrance retention rate (%) *2 |
| Acetylcedrene core T | 8.0 | 100 | |
| Traseolide 100 | 7.5 | 100 | 100 |
| Isolongifolanone | 3.0 | 100 | |
| Coumarin | 0.5 | | |

(continued)

| Fragrance component name | Blending ratio (part by mass) | Evaluation | |
|---|---|---|---|
| | | Encapsulation rate (%) | Fragrance retention rate (%) *2 |
| 2-Phenylethyl alcohol *1 | 10.0 | 86 | |
| Linalool | 10.0 | | |
| Benzylacetate | 4.0 | 89 | |
| Benzylsalicylate | 10.0 | | |
| Citronellol *1 | 10.0 | 94 | 90 |
| Dihydromyrcenol | 10.0 | 90 | 96 |
| Styralyl acetate | 1.0 | 92 | |
| Jasmopyran Forte | 10.0 | | |
| Methyl dihydrojasmonate | 5.0 | 96 | |
| Methyllonone G | 3.0 | 100 | |
| Hexylcinnamic aldehyde | 8.0 | 100 | |
| *1: indicates primary alcohol.<br>*2: indicates a fragrance retention rate one week after start of storage. | | | |

Table 25: Model Fragrance 11 (Example 12)

| Fragrance component name | Blending ratio (part by mass) | Evaluation | |
|---|---|---|---|
| | | Encapsulation rate (%) | Fragrance retention rate (%) |
| Linalool | 22 | 50 | 67 |
| Linalyl acetate | 16 | 67 | 74 |
| Tetrahydrolinalool | 16 | 57 | 88 |
| Caryophyllene | 5 | 84 | 23 |
| Coumarin | 4 | | |
| Eucalyptus oil | 3 | | |
| Isobornyl acetate | 3 | 65 | 83 |
| Ocimene | 3 | 60 | 12 |
| Borneol | 3 | 53 | 77 |
| Neryl acetate | 2 | 71 | 58 |
| alpha pinene | 2 | | |
| Cis-3-hexenol *1 | 2 | 61 | 62 |
| Others | 19 | - | - |
| *1: indicates primary alcohol. | | | |

Table 26: Model Fragrance 10 (Comparative Example 2)

| Fragrance component name | Blending ratio (part by mass) | Evaluation | |
|---|---|---|---|
| | | Encapsulation rate (%) | Fragrance retention rate (%) |
| Acetylcedrenecore T | 8.0 | 13 | 0 |

(continued)

| Fragrance component name | Blending ratio (part by mass) | Evaluation | |
|---|---|---|---|
| | | Encapsulation rate (%) | Fragrance retention rate (%) |
| Traseolide 100 | 7.5 | | |
| Isolongifolanone | 3.0 | 10 | 0 |
| Coumarin | 0.5 | | |
| 2-Phenylethyl alcohol *1 | 10.0 | 3 | 0 |
| Linalool | 10.0 | | |
| Benzylacetate | 4.0 | 2 | 0 |
| Benzylsalicylate | 10.0 | | |
| Citronellol *1 | 10.0 | 3 | 0 |
| Dihydromyrcenol | 10.0 | 2 | 0 |
| Styralyl acetate | 1.0 | 2 | 0 |
| Jasmopyran Forte | 10.0 | | |
| Methyl dihydrojasmonate | 5.0 | 6 | 0 |
| MethylIonone G | 3.0 | 7 | 0 |
| Hexylcinnamic aldehyde | 8.0 | 10 | 0 |
| *1: indicates primary alcohol. | | | |

Table 27 below illustrates results of the constituent component of the shell of the silica capsule, the type of the model fragrance as the organic compound encapsulated in the core, the type and content of primary alcohol in the organic compound, the cLogP value of the primary alcohol, and the encapsulation rate, in Examples and Comparative Examples.

Table 27

| | Silica microcapsule | | | | | | Evaluation |
|---|---|---|---|---|---|---|---|
| | sell | core | | | | | |
| | | | | Primary Alcohol contained in organic compound | | | |
| | Constituent component | Type of organic compound | content of Primary Alcohol in organic compound (mass%) | Type of Primary Alcohol *1 | cLogP | | Encapsulation rate |
| Example 1 | Silica | Model fragrance 1 | 63.1 | Citronellol 42.0% | 3.5 | | 98% |
| | | | | Geraniol 21.1% | 3.3 | | 99% |
| Example 2 | Silica | Model fragrance 2 | 35.0 | 2-Phenylethyl alcohol 10% | 1.6 | | 69% |
| | | | | Tetrahydrogeraniol 25% | 3.6 | | 92% |
| Example 3 | Silica | Model fragrance 3 | 30.0 | Cis-3-hexenol 10% | 1.6 | | 42% |
| | | | | Alcohol C-12 20% | 5.1 | | 100% |

(continued)

| | Silica microcapsule | | | | | Evaluation |
|---|---|---|---|---|---|---|
| | sell | core | | | | |
| | | | Primary Alcohol contained in organic compound | | | |
| | Constituent component | Type of organic compound | content of Primary Alcohol in organic compound (mass%) | Type of Primary Alcohol *1 | cLogP | Encapsulation rate |
| Example 4 | Silica | Model fragrance 4 | 80.0 | Citronellol 80% | 3.5 | 94% |
| Example 5 | Silica | Citronellol | 100.0 | Citronellol 100% | 3.5 | 95% |
| Example 6 | Silica | Model fragrance 5 | 28.0 | Benzyl alcohol 8% | 1.1 | 31% |
| | | | | Cis-3-hexenol 8% | 1.6 | 38% |
| | | | | 2-Phenylethyl alcohol 6% | 1.6 | 80% (total with Linalool) |
| | | | | Geraniol 6% | 3.3 | 88% |
| Example 7 | Silica | Model fragrance 6 | 38.7 | Cis-3-hexenol 0.6% | 1.6 | 62% |
| | | | | Geraniol 12.7% | 3.3 | 95% |
| | | | | Citronellol 25.4% | 3.5 | 90% |
| Example 8 | Silica | Model fragrance 7 | 17.0 | Nerol 17% | 3.7 | 100% |
| Example 9 | Silica | Model fragrance 8 | 12.5 | 6-phenyl-1-hexanol 12.5% | 3.5 | 95% |
| Example 10 | Silica | Model fragrance 9 | 33.3 | 2-Phenylethyl alcohol 30% | 1.6 | 100% |
| | | | | Citronellol 3% | 3.5 | 90% |
| | | | | Cis-3-hexenol 0.3% | 1.6 | (peak cannot be detected) |
| Example 11 | Silica | Model fragrance 10 | 20.0 | 2-Phenylethyl alcohol 10% | 1.6 | 86% (total with Linalool) |
| | | | | Citronellol 10% | 3.5 | 94% |
| Example 12 | Silica | Model fragrance 11 | 2.0 | Cis-3-hexenol 2% | 1.6 | 61% |
| Comparative Example 1 | Polymethacrylate | Model fragrance 1 | 63.1 | Citronellol 42.0% | 3.5 | *2 |
| | | | | Geraniol 21.1% | 3.3 | *2 |
| Comparative Example 2 | Colloidal Silica | Model fragrance 10 | 20.0 | 2-Phenylethyl alcohol 10% | 1.6 | 3% (total with Linalool) |
| | | | | Citronellol 10% | 3.5 | 3% |

*1: numerical value indicates content (mass%) of each primary alcohol in organic compound
*2: since it was determined by an electron microscope that Comparative Example 1 was not encapsulated, no measurement was performed

From Table 27, it can be found that the silica capsules of Examples may encapsulate primary alcohol at a high encapsulation rate as compared to those of Comparative Examples.

Example 13

(Step I)

1.65 g of QUARTAMIN 60W was diluted with 148.43 g of ion-exchanged water to obtain an aqueous phase component. To this aqueous phase component, an oil phase component, which was prepared by mixing 40.01 g of citronellol (cLogP value: 3.5, oil-water interfacial tension: 9.9 mN/m) with 10.01 g of TEOS, was added. The mixed solution was emulsified by using the homomixer under conditions of a rotation speed of 8,000 rpm for 5 min, and a rotation speed of 9,000 rpm for 5 min to obtain an emulsified liquid. At this time, the median diameter $D_{50}$ of the emulsified droplets was 5.2 μm.

After the pH of the obtained emulsified liquid was adjusted to 3.7 by using a 1% by mass sulfuric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (XIII) in which citronellol was encapsulated in amorphous silica.

The encapsulation rate of the fragrance component in the obtained silica capsule (XIII) was measured by the above-described method. The result is noted in Table 28.

Table 28: Citronellol (Example 13)

| Fragrance component name | Blending ratio (part by mass) | Evaluation |
|---|---|---|
| | | Encapsulation rate (%) |
| Citronellol *1 | 100 | 96 |
| *1: indicates primary alcohol. | | |

From Table 28, it can be found that the silica capsules in Example 13 can encapsulate citronellol as primary alcohol at a high encapsulation rate.

(Evaluation of use of silica capsules)

(1) Preparation of softener for evaluation

Silica capsule-containing water dispersions obtained in Example 1 and Comparative Example 3 were added to a softener base having a composition noted in Table 13. Then, a softener for evaluation was prepared. The content of the encapsulated fragrance was set as 0.2% by mass in the softener for evaluation.

(2) Sensory evaluation of scent

Each of four specialized panelists took it home, performed a normal washing machine treatment of a cotton towel by using the evaluation softener by a washing machine equipped with an automatic softener dispenser at each home, and performed a sensory evaluation of scent intensity and scent freshness at the following timings. The sensory evaluation was performed by four specialized panelists with the following evaluation criteria of 0 to 5 (11 grades with a 0.5 increment), and Table 29 illustrates the results as the sums of decided evaluation values.

(Timing of sensory evaluation)

Sample bottle mouth: Smell when the lid of a sample bottle storing the evaluation softener is opened
Dehydrated cloth: Smell of the towel after the washing machine dehydration process is completed
When dehydrated cloth is rubbed: Smell when the towel is rubbed after the washing machine dehydration process is completed
Dry cloth: Smell of the towel after the washing machine-treated towel is dried and drying is completed
When dry cloth is rubbed: Smell when the towel is rubbed after the washing machine-treated towel is dried and drying is completed

(Evaluation criteria)

(Evaluation criteria of scent intensity)

5: Very strong scent
4: Strong scent
3: Easily perceptible scent
2: Scent that is weak to the extent that the type of scent can be recognized (cognitive threshold)
1: Barely perceptible scent (detection threshold)
0: Unscented

(Evaluation criteria of freshnes)

5: Freshness that is very strongly sensed
4: Freshness that is strongly sensed
3: Easily perceptible freshness
2: Weak freshness (cognitive threshold)
1: Barely perceptible freshness (detection threshold)
0: No freshness can be sensed at all

Table 29

|  |  | Example 1 | Comparative Example 3 |
| --- | --- | --- | --- |
| Sample bottle mouth | Fragrance intensity | 16.0 | 17.0 |
|  | Freshness | 15.0 | 7.0 |
| Dehydrated cloth | Fragrance intensity | 11.5 | 9.5 |
|  | Freshness | 13.0 | 5.5 |
| After dehydrated cloth is rubbed | Fragrance intensity | 13.0 | 10.0 |
|  | Freshness | 13.0 | 6.0 |
| Dry cloth | Fragrance intensity | 9.0 | 8.5 |
|  | Freshness | 11.0 | 5.0 |
| After dry cloth is rubbed | Fragrance intensity | 11.0 | 9.5 |
|  | Freshness | 12.0 | 5.0 |

From Table 29, it can be found that since the silica capsules of Example 1 can encapsulate primary alcohol at a high encapsulation rate, even when the silica capsules are used for the washing machine treatment by being blended with the softener, at each timing, the scent intensity is strong, and the freshness can be sufficiently sensed, and thus, the delivery performance of the primary alcohol-containing fragrance for the towel is excellent. From this, it can be found that the silica capsules of the present invention can provide an agent that allows the freshness to be sensed at each timing, and also allows the scent intensity to be strongly sensed.

Example 14

(Step 1)

10.0 g of QUARTAMIN 60W was diluted with 590.7 g of ion-exchanged water to obtain an aqueous phase component. To this aqueous phase component, an oil phase component, which was prepared by mixing 160 g of a model fragrance 13 noted in Table 30 below (cLogP value: 3.6, oil-water interfacial tension: 13.7 mN/m) with 40.3 g of TEOS, was added. The mixed solution was emulsified at room temperature (about 25°C) by using the homomixer under conditions of a rotation speed of 7,000 rpm for 10 min to obtain an emulsified liquid in which the median diameter $D_{50}$ of the emulsified droplets was 1.19 $\mu$m.

After the pH of the obtained emulsified liquid was adjusted to 3.7 by using 0.13 g of a 1% by mass sulfuric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-14) that has a core made of the model fragrance 14 and a first shell made of silica.

(Step 2')

37.5 g of the water dispersion obtained in the step 1 was diluted through addition of 112.5 g of water (dilution ratio 4 times). Then, while the obtained mixed solution was stirred at a liquid temperature of 30°C, 11.8 g of TEOS was added dropwise for 420 min. After the dropping, stirring was further continued for 24 h, and then the mixed solution was cooled so as to form a second shell that encloses the first shell. Then, a water dispersion containing a silica capsule (XIV) in which citronellol was encapsulated in amorphous silica was obtained. The median diameter $D_{50}$ of the silica capsules (XIV) was 4.3 $\mu$m.

The encapsulation rate of the fragrance component in the obtained silica capsule (XIV) was measured by the above-described method. The results are noted in Table 30.

Table 30: Model fragrance 13 (Example 14)

| Fragrance component name | Blending ratio (part by mass) | cLogP | Evaluation |
|---|---|---|---|
| | | | Encapsulation rate (%) |
| Benzylsalicylate | 35 | 4.3 | 98 |
| Iononeα | 5 | 3.9 | 95 |
| Methyl dihydrojasmonate | 20 | 3.0 | 94 |
| Tetrahydrolinalool | 10 | 3.6 | 94 |
| Cis-3-hexenol *1 | 5 | 1.6 | 26 |
| Citronellol *1 | 25 | 3.5 | 92 |
| *1: indicates primary alcohol. | | | |

Comparative Example 4

(Step 1)

10.0 g of QUARTAMIN 60W was diluted with 590.0 g of ion-exchanged water to obtain an aqueous phase component. To this aqueous phase component, an oil phase component, which was prepared by mixing 160.0 g of a model fragrance 14 (cLogP value: 3.8, oil-water interfacial tension: 19.8 mN/m) that is noted in Table 31 below and contains no primary alcohol with 40.0 g of TEOS, was added. The mixed solution was emulsified at room temperature (about 25°C) by using the homomixer at under conditions of a rotation speed of 7,000 rpm for 30 min to obtain an emulsified liquid in which the median diameter $D_{50}$ of the emulsified droplets was 1.60 $\mu$m. Further, 10 minutes after the start of emulsification, the median diameter $D_{50}$ of the emulsified droplets was 1.94 $\mu$m.

After the pH of the obtained emulsified liquid was adjusted to 3.7 by using 0.13 g of a 1% by mass sulfuric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-C4) that has a core made of the model fragrance 15 and a first shell made of silica.

(Step 2)

While 200 g of the water dispersion obtained in the step 1 was stirred at a liquid temperature of 30°C, 11.9 g of TEOS was added dropwise thereto for 420 min. After the dropping, stirring was further continued for 24 h, and then the mixed solution was cooled so as to form a second shell that encloses the first shell. Then, a water dispersion containing a silica capsule (C4) in which the model fragrance 14 was encapsulated in amorphous silica was obtained. The median diameter $D_{50}$ of the silica capsules (C4) was 4.1 μm.

The encapsulation rate of the fragrance component in the obtained silica capsule (C4) was measured by the above-described method. The results are noted in Table 31.

Table 31: Model fragrance 14 (Comparative Example 4)

| Fragrance component name | Blending ratio (part by mass) | cLogP | Evaluation |
|---|---|---|---|
| | | | Encapsulation rate (%) |
| Benzylsalicylate | 50 | 4.3 | 100 |
| Iononeα | 7 | 3.9 | 96 |
| Methyl dihydrojasmonate | 28 | 3.0 | 92 |
| Tetrahydrolinalool | 15 | 3.6 | 91 |

From Table 30, it can be found that encapsulation by the silica capsules of Example 14 can be made at a high encapsulation rate although the primary alcohol is contained.

Further, from the result of the median diameter $D_{50}$ of the emulsified droplets at 10 minutes after the start of emulsification, it can be found that in Example 14, although the emulsification time is a short time, the median diameter $D_{50}$ of the emulsified droplets is significantly reduced as compared to in Comparative Example 4.

In Comparative Example 4, the median diameter $D_{50}$ of the emulsified droplets is decreased with the lapse of time, but the decrease of the median diameter $D_{50}$ is insufficient even after the emulsification time of 30 min has elapsed.

Meanwhile, in Example 14, since the encapsulated organic compound contains a primary alcohol, it can be found that although a shear force is applied at the same rotation speed as in Comparative Example 4, the median diameter $D_{50}$ of the emulsified droplets is reduced in a short time of 10 min as the emulsification time.

From this comparison between Example 14 and Comparative Example 4, it can be found that when the encapsulated organic compound contains a primary alcohol, fine emulsified droplets can be efficiently formed in a shorter time, and the production efficiency of silica capsules having reduced particle sizes can be improved.

In the present invention, the silica capsules encapsulating the primary alcohol-containing organic compound are characterized as silica capsules excellent in the production efficiency because the primary alcohol not only has a function of imparting freshness to the scent, as a fragrance component, but also has a function of promoting emulsification.

Example 15

(Step 1)

1.88 g of QUARTAMIN 60W was diluted with 110.37 g of ion-exchanged water to obtain an aqueous phase component. To this aqueous phase component, an oil phase component, which was prepared by mixing 30 g of a model fragrance 15 (cLogP value: 4.7) noted in Table 32 below with 7.50 g of TEOS, was added. The mixed solution was emulsified at room temperature (about 25°C) by using the homomixer at a rotation speed of 6,500 rpm for 5 min to obtain an emulsified liquid in which the median diameter $D_{50}$ of the emulsified droplets was 2.1 μm.

After the pH of the obtained emulsified liquid was adjusted to 3.64 by using 0.10 g of a 0.2 N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-15) that has a core made of the model fragrance 15 and a shell made of silica.

(Step 2')

While 100 g of the water dispersion obtained in the step 1 was stirred at a liquid temperature of 30°C, 3.0 g of TEOS was added dropwise thereto for 420 min. After the dropping, stirring was further continued for 24 h, and then the mixed solution was cooled so as to form an outer shell that encloses the silica capsule. Then, a water dispersion containing a silica capsule (XV) in which the model fragrance 15 was encapsulated in amorphous silica was obtained. The median diameter $D_{50}$ of the silica capsules (XV) was 2.7 μm.

The encapsulation rate of the fragrance component in the obtained silica capsule (XV) was measured by the above-described method. The results are noted in Table 32.

Table 32: Model fragrance 15 (Example 15)

| Fragrance component name | blending ratio (part by mass) | cLogP | Evaluation |
|---|---|---|---|
| | | | Encapsulation rate (%) |
| Hexyl salicylate | 80 | 5.1 | 100 |
| Tetrahydrogeraniol *1 | 20 | 3.6 | 97 |
| *1: indicates primary alcohol. | | | |

Comparative Example 5

(Step 1)

1.88 g of QUARTAMIN 60W was diluted with 110.34 g of ion-exchanged water to obtain an aqueous phase component. To this aqueous phase component, an oil phase component, which was prepared by mixing 30 g of a model fragrance 16 noted in Table 33 below (cLogP value: 4.8) with 7.51 g of TEOS, was added. The mixed solution was emulsified at room temperature (about 25°C) by using the homomixer at a rotation speed of 6,500 rpm for 5 min to obtain an emulsified liquid in which the median diameter $D_{50}$ of the emulsified droplets was 4.1 μm.

After the pH of the obtained emulsified liquid was adjusted to 3.67 by using 0.02 g of a 0.2N hydrochloric acid aqueous solution, the liquid was transferred to a separable flask equipped with a stirring blade and a cooler. Then, while the liquid temperature was maintained at 30°C, stirring was performed for 24 h to obtain a water dispersion containing a silica capsule (1-C5) that has a core made of the model fragrance 16 and a shell made of silica.

(Step 2')

While 100 g of the water dispersion obtained in the step 1 was stirred at a liquid temperature of 30°C, 2.5 g of TEOS was added dropwise thereto for 420 min. After the dropping, stirring was further continued for 24 h, and then the mixed solution was cooled so as to form an outer shell that encloses the silica capsule. Then, a water dispersion containing a silica capsule (C5) in which the model fragrance 16 was encapsulated in amorphous silica was obtained. The median diameter $D_{50}$ of the silica capsules (C5) was 5.3 μm.

The encapsulation rate of the fragrance component in the obtained silica capsule (XVI) was measured by the above-described method. The results are noted in Table 33.

Table 33: Model fragrance 16 (Comparative Example 5)

| Fragrance component name | blending ratio (part by mass) | cLogP | Evaluation |
|---|---|---|---|
| | | | Encapsulation rate (%) |
| Hexyl salicylate | 80 | 5.1 | 100 |
| Iononeα | 20 | 3.9 | 99 |

From Table 32, it can be found that encapsulation by the silica capsules of Example 15 may be made at a high encapsulation rate although the primary alcohol is contained.

Further, from the result of the median diameter $D_{50}$ of the emulsified droplets at 5 minutes after the start of emulsification, it can be found that in Example 15, although the emulsification time is a short time, the median diameter $D_{50}$ of the emulsified droplets is significantly reduced as compared to in Comparative Example 5.

In Comparative Example 5, the median diameter $D_{50}$ of the emulsified droplets is decreased with the lapse of time, but the decrease of the median diameter $D_{50}$ is insufficient even after the emulsification time of 30 min has elapsed.

Meanwhile, in Example 15, since the encapsulated organic compound contains a primary alcohol, it can be found that although a shear force is applied at the same rotation speed as in Comparative Example 5, the median diameter $D_{50}$ of the emulsified droplets is reduced in a short time of 5 min as the emulsification time.

From this comparison between Example 15 and Comparative Example 5, it can be found that when the encapsulated organic compound contains a primary alcohol, fine emulsified droplets can be efficiently formed in a shorter time, and the production efficiency of silica capsules having reduced particle sizes can be improved.

In the present invention, the silica capsules encapsulating the primary alcohol-containing organic compound are characterized as silica capsules excellent in the production efficiency because the primary alcohol not only has a function of imparting freshness to the scent, as a fragrance component, but also has a function of promoting emulsification.

Further, from Table 32 and Table 33, it can be found that unlike in Comparative Example 5 in which a fragrance not containing a primary alcohol is used as the encapsulated component, in Example 15, the encapsulated component is a fragrance containing tetrahydrogeraniol that is a primary alcohol, and thus emulsified droplets are small even under the same emulsification condition and then the particle sizes of the obtained silica capsules can also be reduced.

INDUSTRIAL APPLICABILITY

According to silica capsules of the present invention, an organic compound containing a primary alcohol can be encapsulated at a high encapsulation rate and can be stably retained for a long period of time even in the formulation containing an oil agent or a surfactant. Therefore, the silica capsules of the present invention can be stably blended in cosmetics, liquid detergents, fabric softeners and the like, and then the delivery performance of the primary alcohol can be satisfactorily exhibited according to various factors such as pressure, humidity, heat, or light. Further, when the primary alcohol contained in the organic compound encapsulated in the core is a fragrance component, its function, that is, the freshness can be imparted to various formulations, and moreover, the present invention is also useful as a production method of silica capsules with a significantly improved production efficiency.

**Claims**

1. A silica microcapsule comprising a shell, and a core comprising one or more organic compounds inside the shell,

    wherein the shell comprises silica as a constituent component, and
    the organic compound comprises a primary alcohol.

2. The silica microcapsule according to claim 1, wherein a content of the primary alcohol in the organic compound is 5% by mass or more and 100% by mass or less.

3. The silica microcapsule according to claim 1 or 2, wherein the organic compound has an oil-water interfacial tension of 3 mN/m or more.

4. The silica microcapsule according to any one of claims 1 to 3, wherein the primary alcohol has 4 or more carbon atoms.

5. The silica microcapsule according to any one of claims 1 to 4, wherein the primary alcohol has a cLogP of 1.0 or more and 7.0 or less.

6. The silica microcapsule according to any one of claims 1 to 5, wherein the primary alcohol is one or more selected from the group consisting of fragrances, antibacterial agents, preservatives, repellents, and active pharmaceutical ingredients.

7. The silica microcapsule according to any one of claims 1 to 6, wherein the primary alcohol is one or more selected from the group consisting of geraniol, citronellol, nellol, 1-dodecanol, tetrahydrogeraniol, cis-3-hexenol, 2-phenylethylalcohol, 6-phenyl-1-hexanol, and benzyl alcohol.

8. The silica microcapsule according to any one of claims 1 to 7, wherein the silica microcapsule has a median diameter $D_{50}$ of 0.1 $\mu$m or more and 100 $\mu$m or less.

9. The silica microcapsule according to any one of claims 1 to 8, wherein the shell comprises silica, which is a hydrolyzed polycondensate of alkoxysilane, as the constituent component.

10. The silica microcapsule according to any one of claims 1 to 8, wherein the shell comprises silica, which is formed and obtained by a sol-gel reaction using alkoxysilane as a precursor, as the constituent component.

11. The silica microcapsule according to any one of claims 1 to 10, wherein the shell has an inner shell that comprises silica, which is a hydrolyzed polycondensate of alkoxysilane, as a constituent component, and an outer shell that comprises silica, which is a hydrolyzed polycondensate of alkoxysilane, as a constituent component, on the outside of the inner shell.

**12.** The silica microcapsule according to any one of claims 1 to 10, wherein the shell comprises silica formed and obtained by performing two sol-gel reaction steps of alkoxysilane, as the constituent component.

**13.** The silica microcapsule according to any one of claims 9 to 12, wherein the alkoxysilane is tetraethoxysilane.

**14.** A softener composition comprising the silica microcapsules according to any one of claims 1 to 13.

**15.** A method of producing a silica microcapsule, which comprises a shell, and a core comprising one or more organic compounds inside the shell,

wherein the shell comprises silica as a constituent component,
the organic compound comprises a primary alcohol, and
the method comprises a step I below.
Step I: subjecting an emulsified liquid obtained by emulsifying an aqueous phase component comprising a cationic surfactant and an oil phase component comprising a primary alcohol-containing organic compound and tetraalkoxysilane, to a sol-gel reaction under an acidic condition, thereby forming a silica capsule that has a core, and a shell whose constituent component is silica, to obtain a water dispersion containing the silica capsule.

**16.** A method of producing silica microcapsule, which comprises a shell, and a core comprising one or more organic compounds inside the shell,

wherein the shell comprises silica as a constituent component,
the organic compound comprises a primary alcohol, and
the method comprises steps 1 and 2 below.

Step 1: subjecting an emulsified liquid obtained by emulsifying an aqueous phase component comprising a cationic surfactant, and an oil phase component comprising an organic compound and tetraalkoxysilane, to a sol-gel reaction under an acidic condition, thereby forming a silica microcapsule (1) that has a core, and a first shell whose constituent component is silica, to obtain a water dispersion containing the silica microcapsule (1).
Step 2: further adding tetraalkoxysilane to the silica microcapsule (1)-containing water dispersion obtained in the step 1, and performing a sol-gel reaction, thereby forming a silica microcapsule having a second shell that encloses the first shell.

**17.** The method according to claim 16, wherein the step 2 is a step 2' below.
Step 2': diluting the silica microcapsule (1)-containing water dispersion obtained in the step 1 through addition of water, and then further adding tetraalkoxysilane and performing a sol-gel reaction, thereby forming a silica microcapsule having a second shell that encloses the first shell.

**18.** The method according to claim 17, wherein in the step 2', a dilution ratio is 2 times or more and 20 times or less.

**19.** The method according to any one of claims 16 to 18, wherein the step 1 comprises steps 1-1 to 1-4 below.

Step 1-1: preparing an aqueous phase component comprising a cationic surfactant.
Step 1-2: preparing an oil phase component by mixing an organic compound with tetraalkoxysilane.
Step 1-3: mixing and emulsifying the aqueous phase component obtained in the step 1-1 and the oil phase component obtained in the step 1-2 to obtain an emulsified liquid.
Step 1-4: subjecting the emulsified liquid obtained in the step 1-3 to a first sol-gel reaction step to form a silica microcapsule (1) that has a core, and a first shell whose constituent component is silica.

## INTERNATIONAL SEARCH REPORT

International application No.

PCT/JP2020/049255

### A. CLASSIFICATION OF SUBJECT MATTER

A61Q 13/00(2006.01)i; A61K 9/10(2006.01)i; A61K 9/50(2006.01)i; A61K 8/04(2006.01)i; A61K 8/11(2006.01)i; A61K 8/25(2006.01)i; A61K 8/34(2006.01)i; A61K 8/891(2006.01)i; A61K 47/02(2006.01)i; A61K 47/10(2006.01)i; A61K 47/34(2017.01)i; B01J 13/14(2006.01)i; C11B 9/00(2006.01)i
FI:     B01J13/14; A61K47/02; A61K8/25; A61K8/34; A61K47/10; A61K8/11; A61K9/50; A61K47/34; A61K8/891; A61K8/04; A61K9/10; A61Q13/00 102; C11B9/00 Z

According to International Patent Classification (IPC) or to both national classification and IPC

### B. FIELDS SEARCHED

Minimum documentation searched (classification system followed by classification symbols)
C11B9/00; A61Q13/00; A61K9/10; A61K9/50; A61K8/04; A61K8/11; A61K8/25; A61K8/34; A61K8/891; A61K47/02; A61K47/10; A61K47/34; B01J13/14

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
Japio-GPG/FX, CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2004-10609 A (INTERNATIONAL FLORAL DESIGN KYOKAI KK) 15 January 2004 (2004-01-15) example 1, paragraph [0020] | 1–8<br>9–19 |
| X<br>A | CN 106148005 A (SOUTHWEST UNIVERSITY) 23 November 2016 (2016-11-23) claim 1, examples 1–3, paragraphs [0034]-[0049] | 1–8<br>9–19 |
| X<br>A | CN 102720054 A (SHANGHAI JIALE CO., LTD.) 10 October 2012 (2012-10-10) claims 1, 3, paragraph [0027] | 1–10, 13<br>11–12, 14–19 |

☒ Further documents are listed in the continuation of Box C.    ☒ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | |
| "O" | document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" document member of the same patent family |

| Date of the actual completion of the international search | Date of mailing of the international search report |
|---|---|
| 03 March 2021 (03.03.2021) | 16 March 2021 (16.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/049255 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | US 2010/0143422 A1 (POPPLEWELL, Lewis Michael) 10 June 2010 (2010-06-10) entire text | 1-19 |
| A | JP 2015-128762 A (KAO CORP.) 16 July 2015 (2015-07-16) entire text | 1-19 |
| A | JP 2003-500428 A (SOL-GEL TECHNOLOGIES LTD.) 07 January 2003 (2003-01-07) entire text | 1-19 |
| A | JP 2015-506816 A (LES INNOVATIONS MATERIUM) 05 March 2015 (2015-03-05) entire text | 1-19 |

Form PCT/ISA/210 (continuation of second sheet) (January 2015)

<table>
<tr><td colspan="2"><b>INTERNATIONAL SEARCH REPORT</b><br>Information on patent family members</td><td colspan="2">International application No.<br>PCT/JP2020/049255</td></tr>
<tr><td>Patent Documents referred in the Report</td><td>Publication Date</td><td>Patent Family</td><td>Publication Date</td></tr>
<tr><td>JP 2004-10609 A</td><td>15 Jan. 2004</td><td>(Family: none)</td><td></td></tr>
<tr><td>CN 106148005 A</td><td>23 Nov. 2016</td><td>(Family: none)</td><td></td></tr>
<tr><td>CN 102720054 A</td><td>10 Oct. 2012</td><td>(Family: none)</td><td></td></tr>
<tr><td>US 2010/0143422 A1</td><td>10 Jun. 2010</td><td>EP 2196257 A2<br>entire text<br>CN 101849891 A<br>MX 2014012659 A<br>ES 2658226 T3</td><td></td></tr>
<tr><td>JP 2015-128762 A</td><td>16 Jul. 2015</td><td>US 2016/0303531 A1<br>entire text<br>WO 2015/083836 A1<br>EP 3078415 A1</td><td></td></tr>
<tr><td>JP 2003-500428 A</td><td>07 Jan. 2003</td><td>US 2002/0037261 A1<br>entire text<br>WO 2000/072806 A2<br>ES 2367701 T3</td><td></td></tr>
<tr><td>JP 2015-506816 A</td><td>05 Mar. 2015</td><td>US 2014/0341958 A1<br>entire text<br>WO 2013/078551 A1<br>CN 104010722 A<br>KR 10-2014-0107352 A<br>CA 2892483 A1</td><td></td></tr>
</table>

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H4265149 A
- US 9532933 B
- JP 2009542667 A
- JP 2012501849 A
- JP 2015128762 A

**Non-patent literature cited in the description**

- **A. LEO.** Comprehensive Medicinal Chemistry. Pergamon Press, 1990, vol. 4, 295